# EUROPÄISCHE PATENTSCHRIFT

(11) **EP 2 301 520 B1**
(45) Veröffentlichungstag und Bekanntmachung des Hinweises auf die Patenterteilung: **08.03.2017**
(21) Anmeldenummer: 10171441.8
(22) Anmeldetag: 30.07.2010
(51) Int. Cl.: A61K 8/44, A61K 8/49, A61Q 5/10, A61Q 5/12

(54) **Oxidative Färbemittel**
Oxidative dye
Colorant oxydatif

(30) Priorität: 17.08.2009 DE 102009028593
(43) Veröffentlichungstag der Anmeldung: 30.03.2011
(73) Patentinhaber: Henkel AG & Co. KGaA, 40589 Düsseldorf (DE)
(72) Erfinder: Zirwen, Sabrina, 22089, Hamburg (DE); Kleen, Astrid, 20457, Hamburg (DE); Rath, Susanne, 20359, Hamburg (DE); Prillwitz, Dörte, 22089, Hamburg (DE)

(56) Entgegenhaltungen:
- EP-A2- 0 873 745
- WO-A1-97/01323
- DE-A1- 4 232 512
- DE-A1- 19 961 910
- DE-A1-102004 062 702
- DATABASE WPI Week 198307 Thomson Scientific, London, GB; AN 1983-16536K XP002736537, & JP S58 4710 A (LION CORP) 11. Januar 1983 (1983-01-11)

## Beschreibung

Die vorliegende Erfindung betrifft Mittel zur Färbung keratinischer Fasern, die eine spezielle Wirkstoffkombination enthalten, ein Verfahren zur Färbung von Haaren mit diesen Mitteln sowie entsprechende Kits in denen die erfindungsgemäßen Mittel konfektioniert werden können.

Für das Färben von Keratinfasern, insbesondere menschlichen Haaren, spielen die sogenannten Oxidationsfärbemittel wegen ihrer intensiven Farben und guten Echtheitseigenschaften eine bevorzugte Rolle. Solche Färbemittel enthalten Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehrerer Kupplerkomponenten die eigentlichen Farbstoffe aus.

In der Vergangenheit wurden häufig Versuche unternommen, die oxidativen Färbemittel hinsichtlich ihrer färberischen Eigenschaften sowie hinsichtlich ihrer pflegenden Eigenschaften zu optimieren.

Bei den färberischen Eigenschaften der oxidativen Färbemittel wird besonderer Wert auf die Erzielung von Färbungen mit guten Echtheitseigenschaften und einer erhöhten Farbkraft gelegt. Insbesondere die Entwicklung von Färbemitteln, die in kürzerer Zeit und/oder mit einer geringeren Farbmenge vergleichbar intensive Farbergebnisse liefern, stand in jüngster Zeit häufig im Mittelpunkt der Untersuchungen.

Bei den pflegenden Eigenschaften ist es besonders wesentlich, dass der Pflegezustand der Haare durch die oxidative Behandlung nicht oder nur in einem möglichst geringen Ausmaß leidet. Hierbei spielt sowohl der Feuchtigkeitshaushalt als auch die innere Struktur der Haare eine wesentliche Rolle. Diese Eigenschaften äußern sich in einem angenehmen Haargefühl, einer guten Kämmbarkeit der Haare im nassen sowie im trockenen Zustand sowie einem schönen Glanz der Haare.

Häufig wurden in der Vergangenheit pflegende Wirkstoffkombinationen entwickelt, die zwar eine Verbesserung des Pflegezustandes der Haare nach der Färbung ermöglichten; dieser Effekt ging dabei jedoch häufig zu Lasten der färberischen Eigenschaften. So wurde teilweise ein deutlich schlechterer Farbaufzug bis hin zu einer unerwünschten Verschiebung der erzielbaren Färbung gefunden.

EP 0 873 745 offenbart Haarfärbemittel mit einer guten Farbtiefe und einer guten Grauabdeckung. Diese Färbemittel enthalten:
(A) eine oder mehrere Keto- und/oder Aldehydverbindungen,
(B) mindestens eine Verbindung, die z.B. aus Oxidationsfarbstoffvorprodukte ausgewählt werden kann; und
(C) einen Farbverstärker ausgewählt, unter anderem, aus Histidin, Pyrrolidon-5-carbonsäure, *etc.*

Aufgabe der vorliegenden Erfindung war es daher, neue Wirkstoffkombinationen für oxidative Färbemittel zu entwickeln, die die gestellten Anforderungen, insbesondere hinsichtlich der färberischen und pflegenden Eigenschaften, in einem besonders hohen Maße erfüllen. Dabei ist es erfindungswesentlich, dass die Verbesserung der pflegenden Eigenschaften nicht zu Lasten der färberischen Eigenschaften der Mittel gehen.

Überraschenderweise wurde nun gefunden, dass eine Wirkstoffkombination aus einer basischen Aminosäure und einer Pyrrolidoncarbonsäure den gestellten Anforderungen in einem besonders hohen Maße genügt, wenn die Mittel gleichzeitig im Wesentlichen frei sind von sauren Aminosäuren. Die erfindungsgemäßen Mittel ermöglichen die Erzeugung satter und intensiver Nuancen mit hoher Farbkraft, wobei die behandelten Fasern nach der Behandlung einen guten Pflegezustand sowie einen angenehmen Feuchtigkeitspegel aufweisen. Außerdem ermöglichen die erfindungsgemäßen Mittel eine bessere Aufhellleistung, eine beschleunigte Farbstoffaufnahme und dementsprechend eine höhere Farbkraft als die Mittel des Standes der Technik.

Ein erster Gegenstand der vorliegenden Erfindung sind daher Mittel zur oxidativen Färbung keratinischer Fasern, insbesondere menschlicher Haare, enthaltend in einem kosmetisch akzeptablen Träger mindestens ein Oxidaionsfarbstoffvorprodukt sowie eine Wirkstoffkombination aus
(a) mindestens einer basischen Aminosäure,
(b) mindestens eine Pyrrolidoncarbonsäure und/oder eines ihrer physiologisch verträglichen Salze,
wobei das Mittel im Wesentlichen frei von sauren Aminosäuren ist.

Unter keratinischen Fasern sind dabei erfindungsgemäß Pelze, Wolle, Federn und insbesondere menschliche Haare zu verstehen. Obwohl die erfindungsgemäßen Oxidationsfärbemittel in erster Linie zum Färben von Keratinfasern geeignet sind, steht prinzipiell einer Verwendung auch auf anderen Gebieten, insbesondere in der Farbphotographie, nichts entgegen.

Als erste erfindungswesentliche Komponente enthalten die Mittel mindestens eine basische Aminosäure. Erfindungsgemäß besonders bevorzugte basische Aminosäuren sind Lysin, Arginin, und Histidin. Arginin und Lysin sind erfindungsgemäß bevorzugt. Arginin ist erfindungsgemäß eine ganz besonders bevorzugte basische Aminosäure.

Es ist erfindungsgemäß bevorzugt, wenn die Mittel die basische Aminosäure in einer Menge von 0,001 bis 1,0 Gew.-%, vorzugsweise von 0,01 bis 0,2 Gew.-%, bezogen auf die anwendungsbereite Mischung enthalten ist.

Als zweite erfindungswesentliche Komponente enthalten die Mittel mindestens eine Pyrrolidoncarbonsäure und/oder eines ihrer physiologisch verträglichen Salze.

Erfindungsgemäß besonders bevorzugte Pyrrolidoncabonsäuren sind die Verbindungen der Formel (I) 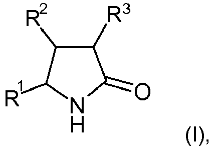 in der
- mindestens einer der Substituenten R¹ bis R³ steht für eine Gruppe -COOR⁴, in der R⁴ Wasserstoff, ein Alkalimetallion, ein Erdalkalimetallion oder ein Ammoniumion ⁺NHR⁵R⁶R⁷, in dem R⁵ bis R⁷ unabhängig voneinander Wasserstoff, Alkylgruppen mit 1 bis 22 C-Atomen, Hydroxyalkylgruppen mit 1 bis 4 C-Atomen, Alkenylgruppen mit 2 bis 22 C-Atomen, Acylgruppen mit 2 bis 22 C-Atomen oder gegebenenfalls substituierte aromatische Gruppen mit 6 bis 10 C-Atomen sind, ist,
   und
- die restlichen Substituenten R¹ bis R³ für Wasserstoff oder Alkylgruppen mit 1 bis 4 C-Atomen stehen.

Die Verbindungen der Formel (I) sind Derivate des 2-Pyrrolidons. Hierbei ist es bevorzugt, wenn genau einer der Susbtituenten R¹ bis R³ für eine Gruppe -COOR⁴ steht, wobei R⁴ wie oben definiert ist, und die restlichen Substituenten R¹ bis R³ für ein Wasserstoffatom stehen.

Besonders bevorzugte Derivate sind die 2-Pyrrolidon-3-carbonsäure, 2-Pyrrolidon-4-carbonsäure und 2-Pyrrolidon-5-carbonsäure und deren Salze. Bevorzugte Salze dieser Verbindungen sind die Natrium-, Kalium-, Calcium-, Magnesium- und solche Ammoniumsalze, bei denen das Ammoniumion neben Wasserstoff eine bis drei (C₁ bis C₄)-Alkylgruppen trägt.

Ganz besonders bevorzugte Verbindungen der Formel (I) werden ausgewählt aus mindestens einer Verbindung der Gruppe, die 2-Pyrrolidon-5-carbonsäure und deren Salze umfasst. Das Natriumsalz ist ganz besonders bevorzugt.

Die erfindungsgemäßen Mittel enthalten die Pyrrolidoncarbonsäure und/oder deren physiologisch verträglichen Salze vorzugsweise in einer Menge von 0,001 bis 1 Gew.-%, insbesondere von 0,01 bis 0,5 Gew.-%, insbesondere von 0,01 bis 0,05 Gew.-%, jeweils bezogen auf die anwendungsbereite Mischung.

Weiterhin hat es sich als erfindungswesentlich erwiesen, wenn die Mittel im Wesentlichen frei von sauren Aminosäuren formuliert sind.

Unter "im Wesentlichen frei von sauren Aminosäure" wird erfindungsgemäß verstanden, dass die Mittel weniger als 0,1Gew.-% an sauren Aminosäuren bezogen auf das anwendungsbereite Mittel enthalten. Mittel, die weniger als als 0,05Gew.-%, insbesondere weniger als 0,01Gew.-% saure Aminosäuren enthalten sind erfindungsgemäß besonders bevorzugt. Ganz besonders bevorzugt ist es, wenn die Mittel vollständig frei von sauren Aminosäuren formuliert sind.

Im Rahmen einer weiteren bevorzugten Ausführungsform hat es sich als vorteilhaft erwiesen, wenn die Mittel weiterhin mindestens eine neutrale Aminosäure enthalten. Erfindungsgemäß bevorzugte neutrale Aminosäuren sind Glyin, Alanin, Valin, Leucin, Isoleucin, Phenylalanin, Tyrosin, Tryptophan und Prolin. Erfindungsgemäß haben sich die aliphatischen neutralen Aminosäuren, insbesondere Glycin, Alanin, Valin, Leucin und Isoleucin als besonders bevorzugt erwiesen. Glycin ist eine ganz besonders bevorzugte neutrale Aminosäure.

Die neutralen Aminosäuren werden vorzugsweise in Mengen von 0,05 bis 2 Gew.-%, insbesondere von 0,01 bis 1 Gew.-%, ganz besonders bevorzugt von 0,1 bis 0,5 Gew.-%, jeweils bezogen auf die anwendungsbereite Mischung, eingesetzt.

Darüberhinaus hat es sich als vorteilhaft erwiesen, wenn das Massenverhältnis von neutraler Aminosäure (nA) zu basischer Aminosäure (bA) nA : bA zwischen 20 und 1 liegt, insbesondere zwischen 18 und 5, ganz besonders zwischen 15 und 10 liegt.

In einer weiteren bevorzugten Ausführungsform der vorliegenden Erfindung enthalten die Mittel mindestens einen Pflanzenextrakt.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Hinsichtlich der erfindungsgemäß bevorzugten Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind.

Erfindungsgemäß sind vor allem die Extrakte aus Moringa Olifeira, Grünem Tee, Eichenrinde, Brennnessel, Hamamelis, Hopfen, Henna, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel, Orchidee bevorzugt.

Besonders bevorzugt sind die Extrakte aus Moringa Olifeira, Grünem Tee, Eichenrinde, Brennnessel, Hamamelis, Hopfen, Kamille, Klettenwurzel, Schachtelhalm, Lindenblüten, Mandel, Aloe Vera, Kokosnuss, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, Ginseng und Ingwerwurzel.

Ganz besonders geeignet sind die Extrakte aus Moringa Olifeira, Grünem Tee, Mandel, Aloe Vera, Kokosnuss, Mango, Aprikose, Limone, Weizen, Kiwi und Melone.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.

Erfindungsgemäß haben sich die Pflanzenextrakte als besonders vorteilhaft erwiesen, die aus einer Pflanze gewonnen wird, die in tropischen und subtropischen Gebieten der Erde kultiviert wird. Insbesondere Pflanzenextrakte, die aus Bestandteilen von Pflanzen der Gattung Moringa, vorzugsweise der Gattung Moringa Oleifera gewonnen werden, sind erfindungsgemäß bevorzugt.

Zur Gattung der Moringagewächse zählen etwa 14 Arten. Eine davon ist Moringa oleifera (Moringa pterygosperma). Weitere Arten sind beispielsweise Moringa drouhardii, Moringa concanensis oder Moringa peregrina. Aus den Samen der Moringagewächse wird durch eine schonende Extraktion mit Wasser und Glycerin das Protein gewonnen. Dieses Protein hat ein Molgewicht von 500 bis 50.000 Dalton. Bevorzugt ist ein Proteinextrakt mit einem Molgewicht von 3000 bis 30.000 Dalton, ganz besonders bevorzugt von 5000 bis 15.000 Dalton. Erfindungsgemäß besonders bevorzugt ist ein Extrakt, der aus der Pflanze Moringa Oleifera gewonnen wird. Weiterhin kann der erfindungsgemäße Extrakt aufgrund der Extraktion selbstverständlich Wasser und Glycerin enthalten. Der Gehalt an extrahiertem Protein im Extrakt beträgt 0,01 bis 20 Gew.%. Ein Gehalt an Protein von 0,01 bis zu 10 Gew.% ist dabei bevorzugt. Besonders bevorzugt ist ein Extrakt mit einem Proteingehalt von 0,01 bis zu 5 Gew.%. Weiterhin sind in dem Extrakt mindestens 30 Gew.% Glycerin enthalten. Schließlich ist Wasser in dem erfindungsgemäßen Extrakt enthalten. Ein derartiges Protein ist beispielsweise unter der Handelsbezeichnung Puricare^{®} LS 9658 von der Fa. Laboratoires Sérobiologiques im Handel erhältlich.

In den erfindungsgemäßen Mitteln ist der zuvor beschriebene Proteinextrakt aus den Samen der Moringagewächse vorzugsweise in einer Menge von mindestens 0,01 bis zu 20 Gew.-% bezogen auf das anwendungbereite Mittel enthalten. Bevorzugt werden Mengen des Extraktes von 0,01 bis zu 10 Gew.-%, ganz besonders bevorzugt Mengen von 0,01 bis 5 Gew.-% bezogen auf die gesamte kosmetische Zusammensetzung verwendet.

Eine weitere Verbesserungen der erfindungsgemäßen Eigenschaften wurde gefunden, wenn die Mittel als alleiniges oder weiteres Alkalsierungsmittel mindestens ein organisches Amin, ausgewählt aus Monoethanolamin, Monoisopropanolamin, 2-Amino-2-methyl-propanol, 2-Amino-2-methyl-1,3-propandiol, 2-Amino-2-ethyl-1,3-propandiol, 2-Amino-2-methylbutanol und Triethanolamin, enthalten. Mittel, die Monoethanolamin und/oder 2-Amino-2-methylpropanol enthalten sind erfindungsgemäß bevorzugt.

Die erfindungsgemäßen Mittel enthalten die genannten organischen Amine vorzugsweise in einer Menge von 0,05 bis 5Gew.-%, insbesondere von 0,1 bis 2Gew.-%, ganz besonders von 0,25 bis 1,5Gew.-%, jeweils bezogen auf das anwendungsbereite Mittel.

Erfindungsgemäß haben sich Mittel als besonders bevorzugt erwiesen, die das genannte organischen Amin in Kombination mit Ammoniak enthalten. Insbesondere die Kombination Ammoniak/Monoethanolamin ist erfindungsgemäß bevorzugt.

In einer weiteren bevorzugten Ausführungsform der vorliegenden Erfindung enthalten die Mittel mindestens ein Phospholipid, wie beispielsweise Sojalecithin, Ei-Lecithin und Kephaline sowie die unter den INCI-Bezeichnungen Linoleamidopropyl PG-Dimonium Chloride Phosphate, Cocamidopropyl PG-Dimonium Chloride Phosphate und Stearamidopropyl PG-Dimonium Chloride Phosphate bekannten Substanzen. Diese werden beispielsweise von der Firma Mona unter den Handelsbezeichnungen Phospholipid EFA^{®}, Phospholipid PTC^{®} sowie Phospholipid SV^{®} vertrieben. Das unter der INCI-Bezeichnung Linoleamidopropyl PG-Dimonium Chloride Phosphate bekannte Phospholipid ist erfindungsgemäß ganz besonders bevorzugt.

Die erfindungsgemäßen Mittel enthalten das mindestens eine Phospholipid vorzugsweise in einer Menge von 0,001 bis 0,5Gew.-%, insbesondere von 0,005 bis 0,1 Gew.-%, ganz besonders von 0,01 bis 0,05Gew.-%, jeweils bezogen auf das anwendungsbereite Mittel.

Erfindungsgemäß bevorzugt sind Mittel, die eine der folgenden Wirkstoffkombinationen enthalten.
- basische Aminosäure + Pyrrolidoncarbonsäure + neutrale Aminosäure
- basische Aminosäure + Pyrrolidoncarbonsäure + Pflanzenextrakt
- basische Aminosäure + Pyrrolidoncarbonsäure + organisches Amin
- basische Aminosäure + Pyrrolidoncarbonsäure + Phospholipid
- basische Aminosäure + Pyrrolidoncarbonsäure + neutrale Aminosäure + Phospholipid
- basische Aminosäure + Pyrrolidoncarbonsäure + Pflanzenextrakt + Phospholipid
- basische Aminosäure + Pyrrolidoncarbonsäure + organisches Amin + Phospholipid
- basische Aminosäure + Pyrrolidoncarbonsäure + neutrale Aminosäure + organisches Amin
- basische Aminosäure + Pyrrolidoncarbonsäure + Pflanzenextrakt + organisches Amin
- basische Aminosäure + Pyrrolidoncarbonsäure + Pflanzenextrakt + neutrale Aminosäure
- basische Aminosäure + Pyrrolidoncarbonsäure + Pflanzenextrakt + organisches Amin + neutrale Aminosäure
- basische Aminosäure + Pyrrolidoncarbonsäure + Pflanzenextrakt + neutrale Aminosäure + Phospholipid
- basische Aminosäure + Pyrrolidoncarbonsäure + Pflanzenextrakt + organisches Amin + Phospholipid
- basische Aminosäure + Pyrrolidoncarbonsäure + Pflanzenextrakt + organisches Amin + Phospholipid + neutrale Aminosäure

Bei dieser Aufzählung gelten die oben genannte Definitionen sowie Bevorzugungen.

Die folgenden Kombinationen sind ganz besonders bevorzugt:
- Arginin + Salz der 2-Pyrrolidon-5-carbonsäure + Glycin
- Arginin + Salz der 2-Pyrrolidon-5-carbonsäure + Moringaextrakt
- Arginin + Salz der 2-Pyrrolidon-5-carbonsäure + Monoethanolamin
- Arginin + Salz der 2-Pyrrolidon-5-carbonsäure + Phospholipid
- Arginin + Salz der 2-Pyrrolidon-5-carbonsäure + Glycin + Phospholipid
- Arginin + Salz der 2-Pyrrolidon-5-carbonsäure + Moringaextrakt + Phospholipid
- Arginin + Salz der 2-Pyrrolidon-5-carbonsäure + Monoethanolamin + Phospholipid
- Arginin + Salz der 2-Pyrrolidon-5-carbonsäure + Glycin + Monoethanolamin
- Arginin + Salz der 2-Pyrrolidon-5-carbonsäure + Moringaextrakt + Monoethanolamin
- Arginin + Salz der 2-Pyrrolidon-5-carbonsäure + Moringaextrakt + Glycin
- Arginin + Salz der 2-Pyrrolidon-5-carbonsäure + Moringaextrakt + Monoethanolamin + Glycin
- Arginin + Salz der 2-Pyrrolidon-5-carbonsäure + Moringaextrakt + Glycin + Phospholipid
- Arginin + Salz der 2-Pyrrolidon-5-carbonsäure + Moringaextrakt + Monoethanolamin + Phospholipid
- Arginin + Salz der 2-Pyrrolidon-5-carbonsäure + Moringaextrakt + Monoethanolamin + Phospholipid + Glycin

Als weiteren erfindungswesentlichen Bestandteil enthalten die erfindungsgemäßen Färbemittel mindestens ein Oxidationsfarbstoffvorprodukt. Als Oxidationsfarbstoffvorprodukt enthalten die Mittel mindestens eine Entwicklerkomponente sowie gegebenenfalls mindestens eine Kupplerkomponente.

Es kann erfindungsgemäß bevorzugt sein, als Entwicklerkomponente ein p-Phenylendiaminderivat oder eines seiner physiologisch verträglichen Salze einzusetzen.
Besonders bevorzugte p-Phenylendiamine sind ausgewählt aus p-Phenylendiamin, p-Toluylendiamin, 2-Chlor-p-phenylendiamin, N,N-Bis-(2-hydroxyethyl)-p-phenylendiamin und N-Phenyl-p-phenylendiamin, 2-(2-Hydroxyethyl)-p-phenylendiamin, sowie ihren physiologisch verträglichen Salzen.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.
Bevorzugte zweikernige Entwicklerkomponenten sind insbesondere: N,N'-Bis-(2-hydroxyethyl)-N,N'-bis-(4'-aminophenyl)-1,3-diamino-propan-2-ol und Bis-(2-hydroxy-5-aminophenyl)-methan und deren physiologisch verträglichen Salze.

Weiterhin kann es erfindungsgemäß bevorzugt sein, als Entwicklerkomponente ein p-Aminophenotderivat oder eines seiner physiologisch verträglichen Salze einzusetzen.
Bevorzugte p-Aminophenole sind insbesondere p-Aminophenol, N-Methyl-p-aminophenol, und 4-Amino-3-methyl-phenol sowie deren physiologisch verträglichen Salze.

Ferner kann die Entwicklerkomponente ausgewählt sein aus o-Aminophenol und seinen Derivaten, wie beispielsweise 2-Amino-5-methylphenol und dessen physiologisch verträglichen Salze.

Weiterhin kann die Entwicklerkomponente ausgewählt sein aus heterocyclischen Entwicklerkomponenten, wie beispielsweise den Pyridin-, Pyrimidin-, Pyrazol-, Pyrazol-Pyrimidin-Derivaten und ihren physiologisch verträglichen Salzen.

Bevorzugte Pyrimidin-Derivate sind insbesondere 2,4,5,6-Tetraaminopyrimidin und 4-Hydroxy-2,5,6-triaminopyrimidin und deren physiologisch verträglichen Salze.

Ein bevorzugtes Pyrazol-Derivat ist 4,5-Diamino-1-(2-hydroxyethyl)-pyrazol und dessen physiologisch verträglichen Salze.

Ganz besonders bevorzugte Entwicklerkomponenten werden ausgewählt, aus mindestens einer Verbindung aus der Gruppe, die gebildet wird aus p-Phenylendiamin, p-Toluylendiamin, 2-(β-Hydroxyethyl)-p-phenylendiamin, 2-Methoxymethyl-p-phenylendiamin, 2-(α,β-Dihydroxyethyl)-p-phenylendiamin, N,N-Bis-(β-hydroxyethyl)-p-phenylendiamin, N-(4-Amino-3-methylphenyl)-N-[3-(1H-imidazol-1-yl)propyl]amin, N,N'-Bis-(β-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(α,β-dihydroxyethyl)-phenol und 4-Amino-2-(diethylaminomethyl)-phenol, 4,5-Diamino-1-(β-hydroxyethyl)-pyrazol, 2,4,5,6-Tetraaminopyrimidin, 4-Hydroxy-2,5,6-triaminopyrimidin, 2-Hydroxy-4,5,6-triaminopyrimidin, sowie den physiologisch verträglichen Salzen dieser Verbindungen.

Kupplerkomponenten im Sinne der Erfindung erlauben mindestens eine Substitution eines chemischen Restes des Kupplers durch die oxidierte Form der Entwicklerkomponente. Dabei bildet sich eine kovalente Bindung zwischen Kuppler- und Entwicklerkomponente aus. Kuppler sind bevorzugt zyklische Verbindungen, die am Zyklus mindestens zwei Gruppen tragen, ausgewählt aus (i) gegebenenfalls substituierten Aminogruppen und/oder (ii) Hydroxygruppen. Wenn die zyklische Verbindung ein Sechsring (bevorzugt aromatisch) ist, so befinden sich die besagten Gruppen bevorzugt in ortho-Position oder meta-Position zueinander.

Erfindungsgemäße Kupplerkomponenten werden bevorzugt als mindestens eine Verbindung aus einer der folgenden Klassen ausgewählt:
- m-Aminophenol und/oder dessen Derivate,
- m-Diaminobenzol und/oder dessen Derivate,
- o-Diaminobenzol und/oder dessen Derivate,
- o-Aminophenolderivate, wie beispielsweise o-Aminophenol,
- Naphthalinderivate mit mindestens einer Hydroxygruppe,
- Di- beziehungsweise Trihydroxybenzol und/oder deren Derivate,
- Pyridinderivate,
- Pyrimidinderivate,
- Monohydroxyindol-Derivate und/oder Monoaminoindol-Derivate,
- Monohydroxyindolin-Derivate und/oder Monoaminoindolin-Derivate,
- Pyrazolonderivate, wie beispielsweise 1-Phenyl-3-methylpyrazol-5-on,
- Morpholinderivate wie beispielsweise 6-Hydroxybenzomorpholin oder 6-Amino-benzomorpholin,
- Chinoxalinderivate wie beispielsweise 6-Methyl-1,2,3,4-tetrahydrochinoxalin,
Gemische aus zwei oder mehrer Verbindungen aus einer oder mehrerer dieser Klassen sind im Rahmen dieser Ausführungsform ebenso erfindungsgemäß.

Als Kupplerkomponenten werden in der Regel m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, 1,5- und 2,7-Dihydroxynaphthalin, 1-Acetoxy-2-methoxynaphthalin, Resorcin, 4-Chlor-resorcin und 2-Amino-3-hydroxypyridin und deren physiologisch verträglichen Salze.

Erfindungsgemäß bevorzugte Kupplerkomponenten sind
(A) m-Aminophenol und dessen Derivate wie beispielsweise 5-Amino-2-methylphenol, 3-Amino-2-chlor-6-methylphenol, 5-Amino-4-chlor-2-methylphenol, 5-(2'-Hydroxyethyl)-amino-2-methylphenol und 2,4-Dichlor-3-aminophenol,
(B) o-Aminophenol und dessen Derivate, beispielsweise 2-Amino-5-ethylphenol,
(C) m-Diaminobenzol und dessen Derivate wie beispielsweise 2,4-Diaminophenoxy-ethanol, 1,3-Bis-(2',4'-diaminophenoxy)-propan, 1-Methoxy-2-amino-4-(2'-hydroxyethylamino)benzol, 2,6-Bis-(2'-hydroxyethylamino)-1-methylbenzol,
(D) o-Diaminobenzol und dessen Derivate,
(E) Di- beziehungsweise Trihydroxybenzolderivate wie beispielsweise 2-Methylresorcin und 1,2,4-Trihydroxybenzol,
(F) Pyridinderivate wie beispielsweise 3-Amino-2-methylamino-6-methoxypyridin, 2,6-Diaminopyridin, 2,6-Dihydroxy-3,4-dimethylpyridin und 3,5-Diamino-2,6-dimethoxypyridin,
(G) Naphthalinderivate wie beispielsweise 1-Naphthol und 2-Methyl-1-naphthol,
(H) Morpholinderivate wie beispielsweise 6-Hydroxybenzomorpholin,
(I) Chinoxalinderivate,
(J) Pyrazolderivate wie beispielsweise 1-Phenyl-3-methylpyrazol-5-on,
(K) Indolderivate wie beispielsweise 6-Hydroxyindol,
(L) Pyrimidinderivate oder
(M) Methylendioxybenzolderivate wie beispielsweise 1-(2'-Hydroxyethyl)-amino-3,4-methylendioxybenzol
sowie deren physiologisch verträglichen Salze.

Erfindungsgemäß besonders bevorzugte Kupplerkomponenten sind 1-Naphthol, 1,5- und 2,7-Dihydroxynaphthalin, 5-Amino-2-methylphenol, 2-Amino-3-hydroxypyridin, Resorcin, 4-Chlorresorcin, 2-Methylresorcin und 2,6-Dihydroxy-3,4-dimethylpyridin und deren physiologisch verträglichen Salze. 2-Amino-3-hydroxypyridin und 3-Amino-2-methylamino-6-methoxypyridin haben sich als erfindungsgemäß ganz besonders bevorzugte Kupplerkomponenten erwiesen.

Die erfindungsgemäßen oxidativen Färbemittel enthalten sowohl die Entwicklerkomponenten als auch die Kupplerkomponenten bevorzugt in einer Menge von 0,005 bis 20 Gew.-%, vorzugsweise 0,1 bis 5 Gew.-%, jeweils bezogen auf das gesamte Oxidationsfärbemittel. Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuß einzelner Oxidationsfarbstoffvorprodukte nicht nachteilig, so daß Entwicklerkomponenten und Kupplerkomponenten in einem Mol-Verhältnis von 1:0,5 bis 1:3, insbesondere 1:1 bis 1:2, enthalten sein können.

In einer weiteren Ausführungsform der vorliegenden Erfindung können die Färbemittel mindestens eine Vorstufe eines naturanalogen Farbstoffs enthalten.

Als Farbstoffvorstufen naturanaloger Farbstoffe werden bevorzugt solche Indole und Indoline eingesetzt, die mindestens zwei Gruppen ausgewählt aus Hydroxy- und/oder oder Aminogruppen, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer weiteren Ausführungsform enthalten die Färbemittel mindestens ein Indol- und/oder Indolinderivat. Erfindungsgemäße Zusammensetzungen, die Vorstufen naturanaloger Farbstoffe enthalten, werden bevorzugt als luftoxidative Färbemittel verwendet. In dieser Ausführungsform werden die besagten Zusammensetzungen folglich nicht mit einem zusätzlichen Oxidationsmittel versetzt.

Besonders bevorzugte Derivate des Indolins sind das 5,6-Dihydroxyindolin und das 2,3-Dioxoindolin (Isatin) und deren physiologisch verträglichen Salze.
Ein besonders bevorzugtes Derivat des Indols ist das 5,6-Dihydroxyindol und dessen physiologisch verträglichen Salze.

Die Indolin- beziehungsweise die Indol-Derivate können sowohl als freie Basen als auch in Form ihrer physiologisch verträglichen Salze mit anorganischen oder organischen Säuren, z. B. der Hydrochloride, der Sulfate und Hydrobromide, eingesetzt werden.

Die Indolin- und Indol-Derivate können in den erfindungsgemäßen Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden. Die Indol- oder Indolin-Derivate sind in diesen üblicherweise in Mengen von 0,05-10 Gew.-%, vorzugsweise 0,2-5 Gew.-% enthalten.

In einer weiteren Ausführungsform kann es erfindungsgemäß bevorzugt sein, das Indolin- oder Indolderivat in Färbemitteln in Kombination mit mindestens einer Aminosäure oder einem Oligopeptid einzusetzen. Die Aminosäure ist vorteilhafterweise eine α-Aminosäure; ganz besonders bevorzugte α-Aminosäuren sind Arginin, Ornithin, Lysin, Serin und Histidin, insbesondere Arginin.

Neben den erfindungsgemäßen p-Phenylendiaminderivaten können die erfindungsgemäßen Färbemittel in einer weiteren bevorzugten Ausführungsform der vorliegenden Erfindung zur Nuancierung einen oder mehrere direktziehende Farbstoffe enthalten. Dabei handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole.

Direktziehende Farbstoffe können in anionische, kationische und nichtionische direktziehende Farbstoffe unterteilt werden.

Als anionische direktziehende Farbstoffe eignen sich insbesondere 2,4-Dinitro-1-naphthol-7-sulfonsäure-dinatriumsalz (C.I. 10,316; Acid Yellow 1; Food Yellow No. 1), 2-(Indan-1,3-dion-2-yl)chinolin-x,x-sulfonsäure (Gemisch aus Mono- und Disulfonsäure) (C.I. 47,005; D&C Yellow No. 10; Food Yellow No. 13; Acid Yellow 3, Yellow 10), 5-Hydroxy-1-(4-sulfophenyl)-4-[(4-sulfophenyl)-azo]pyrazol-3-carbonsäure-trinatriumsalz (C.I. 19,140; Food Yellow No. 4; Acid Yellow 23), 3-[(4-Phenylamino)phenyl]azobezolsulfonsäuresäure-natriumsalz (C.I. 13,065; Ki406; Acid Yellow 36), 4-[(2-Hydroxynaphth-1-yl)azo]-benzolsulfonsäure-natriumsalz (C.I. 15,510; Acid Orange 7), 6-Hydroxy-5-[(4-sulfonaphth-1-yl)azo]-2,4-naphthalin-disulfonsäure-trinatriumsalz (C.I. 16,255; Ponceau 4R; Acid Red 18), 8-Amino-1-hydroxy-2-(phenylazo)-3,6-naphthalin-disulfonsäure-dinatriumsalz (C.I. 17,200; Acid Red 33; Red 33), N-[6-(Diethylamino)-9-(2,4-disulfophenyl)-3H-xanthen-3-yliden]-N-ethylethanammonium-hydroxid, inneres Salz, Natriumsalz (C.I. 45,100; Acid Red 52), 2',4',5',7'-Tetrabrom-4,5,6,7-tetrachlor-3',6'- dihydroxyspiro[isobenzofuran-1(3H),9'[9H]xanthen]-3-on-dinatriumsalz (C.I. 45,410; Acid Red 92), 3-Hydroxy-4-[(4-methyl-2-sulfonphenyl)azo]-2-naphthalincarbonsäure-calciumsalz (C.I. 15,850:1; Pigment Red 57:1), 1,4-Bis[(2-sulfo-4-methylphenyl)amino]-9,10-anthrachinon-dinatriumsalz (C.I. 61,570; Acid Green 25), Bis[4-(dimethylamino)phenyl]-(3,7-disulfo-2-hydroxynaphth-1-yl)carbenium-inneres Salz, Natriumsalz (C.I. 44,090; Food Green No. 4; Acid Green 50), N-[4-[(2,4-Disulfophenyl)[4-[ethyl(phenylmethyl)amino)phenyl]methylen]-2,5-cyclohexadien-1-yliden]-N-ethylbenzolmethanaminium-hydroxid, inneres Salz, Natriumsalz (C.I. 42,080; Acid Blue 7), (2-Sulfophenyl)di[4-(ethyl((4-sulfophenyl)methyl)amino)phenyl]-carbenium-dinatriumsalz Betain (C.I. 42,090; Acid Blue 9; FD&C Blue No. 1), 1-Amino-4-(cyclohexylamino)-9,10-anthrachinon-2-sulfonsäure-natriumsalz (C.I. 62045; Acid Blue 62), 1-Hydroxy-4-[(4-methyl-2- sulfophenyl)amino]-9,10-anthrachinon-natriumsalz (C.I. 60,730; D&C Violett No. 2; Acid Violet 43), 5-Amino-4-hydroxy-6-[(4-nitrophenyl)-azo]-3-(phenylazo)-2,7-naphthalin-disulfonsäure-dinatriumsalz (C.I. 20,470; Acid Black 1), 3-Hydroxy-4-[(2-hydroxynaphth-1-yl)azo]-7-nitro-1-naphthalin-sulfonsäure-chromkomplex (3:2) (C.I. 15,711; Acid Black 52), 3',3",4,5,5',5",6,7-Octabromphenolsulfonphthalein (Tetrabromphenolblau).

Bevorzugte anionische direktziehende Farbstoffe sind die unter den internationalen Bezeichnungen bzw. Handelsnamen Acid Yellow 1, Acid Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1 und Acid Black 52 bekannten Verbindungen.

Als kationische direktziehende Farbstoffe eignen sich insbesondere Di[4-(diethylamino)phenyl][4-(ethylamino)naphthyl]carbenium-chlorid (C.I. 42,595; Basic Blue 7), Di[4-(dimethylamino)phenyl][4-(phenylamino)naphthyl] carbenium-chlorid (C.I. 44,045; Basic Blue 26), 8-Amino-2-brom-5-hydroxy-4-imino-6-[(3-(trimethylammonio)phenyl)amino]-1(4H)-naphthalinon-chlorid (C.I. 56,059; Basic Blue No. 99), Tri(4-amino-3-methylphenyl)carbenium-chlorid (C.I. 42,520; Basic Violet 2), Di(4-aminophenyl)(4-amino-3- methylphenyl)carbeniumchlorid (C.I. 42,510 Basic Violet 14), 1-[(4-Aminophenyl)azo]-7-(trimethylammonio)- 2-naphthol-chlorid (C.I. 12,250; Basic Brown 16), 1-[(4-Amino-3-nitrophenyl)azo]-7-(trimethylammonio)-2-naphthol-chlorid (C.I. 12,251; Basic Brown 17), 3-[(4-Amino-2,5-dimethoxyphenyl)azo]-N,N,N-trimethylbenzolaminium-chlorid (C.I. 12,605, Basic Orange 69), 2-[((4-Dimethylamino)phenyl)azo]-1,3-dimethyl-1H-imidazoliumchlorid (Basic Red 51), 2-Hydroxy-1-[(2-methoxyphenyl)azo]-7-(trimethylammonio)-naphthalin-chlorid (C.I. 12,245; Basic Red 76), 2-[4-Aminophenyl]azo]-1,3-dimethyl-1H-Imidazolium-chlorid (Basic Orange 31), 3-Methyl-1-phenyl-4-[(3-(trimethylammonio)phenyl)azo]-pyrazol-5-on-chlorid (C.I. 12,719; Basic Yellow 57), 1-Methyl-4-((methylphenylhydrazono)methyl)-pyridinium-methylsulfat (Basic Yellow 87), 1-(2-Morpholiniumpropylamino)-4-hydroxy-9,10-anthrachinon-methylsulfat, 4-Formyl-1-methylquinolonium-p-toluensulfonat und direktziehende Farbstoffe, die einen Heterocyclus enthalten, der mindestens ein quaternäres Stickstoffatom aufweist.

Als nichtionische direktziehende Farbstoffe eignen sich insbesondere nichtionische Nitro- und Chinonfarbstoffe und neutrale Azofarbstoffe.

Geeignete blaue Nitrofarbstoffe sind insbesondere 1,4-Bis[(2-hydroxyethyl)amino]-2-nitrobenzol (HC Violet BS), 1-(2-Hydroxyethyl)amino-2-nitro-4-[di(2-hydroxyethyl)amino]-benzol (HC Blue 2), 4-[Di(2-hydroxyethyl)amino]-1-[(2-methoxyethyl)amino]-2-nitrobenzol (HC Blue 11), 4-[Ethyl-(2-hydroxyethyl)-amino]-1-[(2-hydroxyethyl)amino]-2-nitrobenzol-hydrochlorid (HC Blue 12), 1-(2-Hydroxyethyl)amino-2-nitro-4-N-ethyl-N-(2-hydroxyethyl)aminobenzol (HC Blue 15), 1-Amino-3-methyl-4-[(2-hydroxyethyl)amino]-6-nitrobenzol (HC Violet 1), 1-(3-Hydroxypropylamino)-4-[di(2-hydroxyethyl)amino]-2-nitrobenzol (HC Violet 2).

Geeignete rote Nitrofarbstoffe sind insbesondere 1-Amino-4-[(2-hydroxyethyl)amino]-2-nitrobenzol (HC Red 7), 2-Amino-4,6-dinitrophenol (Pikraminsäure) und deren Salze, 1,4-Diamino-2-nitrobenzol (C.I. 76,070), 4-Amino-2-nitro-diphenylamin (HC Red 1), 1-Amino-4-[di(2-hydroxyethyl)amino]-2-nitrobenzol-hydrochlorid (HC Red 13), 1-Amino-4-[(2-hydroxyethyl)-amino]-5-chlor-2-nitrobenzol, 4-Amino-1-[(2-hydroxyethyl)amino]-2-nitrobenzol (HC Red 3), 4-[(2-Hydroxyethyl)-amino]-3-nitrotoluol, 4-Amino-3-nitrophenol, 4-[(2-Hydroxyethyl)-amino]-3-nitrophenol, 4-[(2-Nitrophenyl)amino]phenol (HC Orange 1), 1-[(2-Aminoethyl)amino]-4-(2-hydroxyethoxy)-2-nitrobenzol (HC Orange 2), 1-Amino-5-chlor-4-[(2,3-dihydroxypropyl)amino]-2-nitrobenzol (HC Red 10), 5-Chlor-1,4-[di(2,3-dihydroxypropyl)amino]-2-nitrobenzol (HC Red 11), 2-[(2- Hydroxyethyl)amino]-4,6-dinitrophenol und deren Salze, 4-Ethylamino-3-nitrobenzoesäure, 2-[(4-Amino-2-nitrophenyl)amino]-benzoesäure, 2-Chlor-6-ethylamino-4-nitrophenol, 2-Amino-6-chlor-4-nitrophenol, 4-[(3-Hydroxypropyl)amino]-3-nitrophenol (HC Red BN), 1,2,3,4-Tetrahydro-6-nitrochinoxalin, 6-Hydroxy-5-((2-methoxy-5-methyl-4-sulfophenyl)azo)- 2-naphthalensulfonsäure (Curry Red).

Geeignete gelbe Nitrofarbstoffe sind insbesondere 1,2-Diamino-4-nitrobenzol (C.I. 76,020), 1-[(2-Hydroxyethyl)amino]-2-nitrobenzol (HC Yellow 2), 1-(2-Hydroxyethoxy)-2-[(2-hydroxyethyl)amino]-5-nitrobenzol (HC Yellow 4), 1-Amino-2-[(2-hydroxyethyl)amino]-5-nitrobenzol (HC Yellow 5), 4-[(2,3-Dihydroxypropyl)-amino]-3-nitro-1-trifluormethyl-benzol (HC Yellow 6), 2-[(2- Hydroxyethyl)-amino]-1-methoxy-5-nitrobenzol, 2-Amino-4-nitrophenol, 1-(2-Hydroxyethoxy)-3-methylamino-4-nitrobenzol, 2,3-(Dihydroxypropoxy)-3-methylamino-4-nitrobenzol, 3-[(2-Aminoethyl)amino]-1-methoxy-4-nitrobenzol-hydrochlorid (HC Yellow 9), 1-Chlor-2,4-bis[(2-hydroxyethyl)amino]-5-nitrobenzol (HC Yellow 10), 2-[(2-Hydroxyethyl)amino]-5-nitrophenol (HC Yellow 11), 1-[(2'-Ureidoethyl)amino]-4-nitrobenzol, 1-Amino-4-[(2-aminoethyl)amino]-5-methyl-2-nitrobenzol, 4-[(2-Hydroxyethyl)amino]-3-nitro-1-methylbenzol, 1-Chlor-4-[(2-hydroxyethyl)amino]-3-nitrobenzol (HC Yellow 12), 4-[(2-Hydroxyethyl)amino]-3-nitro-1-trifluormethyl-benzol (HC Yellow 13).

Geeignete Chinonfarbstoffe sind insbesondere 1-[(2-Hydroxyethyl)amino]-4-methylamino-9,10-anthrachinon (C.I. 61,505, Disperse Blue 3), Mischungen aus 1,4-bis[(2-hydroxyethyl)amino]anthra-9,10-quinon mit 1-[(2-hydroxyethyl)amino]-4-[(3-hydroxypropyl)amino]anthra-9,10-quinon und 1,4-bis[(3-hydroxypropyl)amino]anthra-9,10-quinone (Disperse Blue 377), 1,4-Diamino-9,10-anthrachinon (C.I. 61,100, Disperse Violet 1), 1-Amino-4-(methylamino)-9,10-anthrachinon (C.I. 61,105, Disperse Violet 4, Solvent Violet No. 12), 2-Hydroxy-1,4-naphthochinon (Lawsone, C.I. 75,480, Natural Orange 6), 1,4-bis[(2,3-dihydroxypropyl)amino]-9,10-anthracenedion (HC Blue 14).

Geeignete neutrale Azofarbstoffe sind insbesondere 1-[Di(2-hydroxyethyl)amino]-3-methyl-4-[(4-nitrophenyl)azo]-benzol (C.I. 11,210, Disperse Red 17), 1-[Di(2-hydroxyethyl)amino]-4-[(4-nitrophenyl)azo]-benzol (Disperse Black 9), 4-[(4- Aminophenyl)azo]-1-[di(2-hydroxyethyl)amino]-3-methylbenzol (HC Yellow 7), 2,6-Diamino-3-[(pyridin-3-yl)azo]-pyridin, 4-[(4-Nitrophenyl)azo]-anilin (C.I. 11,005; Disperse Orange 3).

Es ist nicht erforderlich, dass die direktziehenden Farbstoffe jeweils einheitliche Verbindungen darstellen. Vielmehr können, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Die direktziehenden Farbstoffe werden jeweils bevorzugt in einer Menge von 0,001 bis 20 Gew.-%, bezogen auf die gesamte Anwendungszubereitung, eingesetzt. Die Gesamtmenge an direktziehenden Farbstoffen beträgt vorzugsweise höchstens 20 Gew.-%.

Weiterhin können als direktziehende Farbstoffe auch Naturfarbstoffe eingesetzt werden. Als Naturfarbstoffe im Sinne der Erfindung sind Farbstoffe zu verstehen, die aus Pflanzenteilen gewonnen werden. Dazu eignen sich bevorzugt unterschiedliche Pflanzenteile, je nach gewünschtem Farbstoff. Es versteht sich jedoch, dass ein Farbstoff aus mehr als einem Pflanzenteil gewonnen werden kann.

Die bevorzugten Pflanzenteile zur Gewinnung von Naturfarbstoffen sind in der Gruppe enthalten, die gebildet wird aus Wurzel, Stengel, Rinde, Kernholz, Harz, Blüte, Blatt, Frucht und Pflanzensaft. Im Sinne der Erfindung ist es für Naturfarbstoffe weiterhin ist nicht erforderlich, dass die aus den Pflanzenteilen gewonnenen Mittel Farbstoffe als solche darstellen, sondern dass sie gegebenenfalls auch Farbstoffvorstufen darstellen, die durch nachfolgende chemische Reaktionen den eigentlichen Farbstoff ausbilden. Genannt sei hier insbesondere die Oxidation mit Luftsauerstoff zur Ausbildung des Chromophors.

Es ist insbesondere im Sinne der Erfindung nicht erforderlich, dass die Farbstoffe beziehungsweise Farbstoffvorstufen jeweils einheitliche Verbindungen darstellen. Bedingt durch unterschiedliche, nicht einheitliche Gewinnungsverfahren wie beispielsweise unterschiedliche Extraktions- oder Fermentationsbedingungen oder durch Nutzung verschiedener, regionaler Unterarten und verschiedener Pflanzenteile oder teilweise auch aus kultur-historischer Tradition enthalten manche genannten Naturfarbstoffe bewusst keine einheitlichen Verbindungen, sondern Gemische aus Farbstoffen, Farbstoffvorstufen und weiteren Komponenten, wie insbesondere Gerbstoffen.

Im Sinne der Erfindung als Naturfarbstoff wird selbstverständlich auch eine aus einem solchen Gemisch isolierte, diskrete farbegebende Verbindung oder gegebenenfalls auch mehrere, aus einem solchen Gemisch isolierte, diskrete, farbgebende Verbindungen verstanden. Detaillierte Beschreibungen der Naturfarbstoffe finden sich unter anderem in der dem Fachmann bekannten Handbuch der Naturfarbstoffe: Vorkommen, Verwendung, Nachweis, H. Schweppe, ecomed Verlagsgesellschaft, 1993, Landsberg/Lech.

Folgende Klassen von Naturfarbstoffen finden in den erfindungsgemäßen Mitteln als Färbemittel für keratinische Fasern bevorzugte Anwendung:
a) Diaryloylmethanfarbstoffe,
   wie beispielsweise in Kurkuma (Gelbwurzel) enthalten (C.I. Natural Yellow 3) - *Curcuma domestica* mit den färbenden Inhaltsstoffen Curcumin (C.I. 75300), Demethoxycurcumin und Bisdemethoxycurcumin. 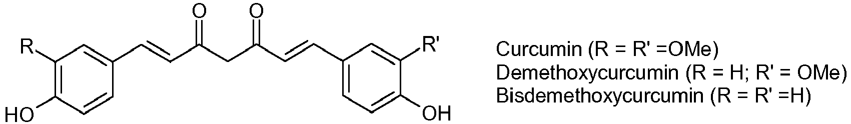
b) Naphthochinonfarbstoffe,
   wie beispielsweise in Henna, rot (C.I. Natural Orange 6) aus den Blättern des Hennastrauchs - *Lawsonia inermis L.* mit dem färbenden Inhaltsstoff Lawson (C.I. 75480): 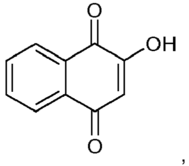 in Fruchtschalen und Blättern (C.I. Natural Brown 7) des Walnussbaums - *Juglus regia L.* mit dem färbenden Inhaltsstoff Juglon (C.I. 75500): 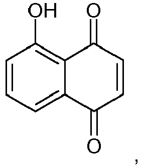 in Alkannawurzel oder Shikoninwurzel (C.I. Natural Red 20) - *Alkanna tinctoria* beziehungsweise *Lithospermum erythrorhizon* mit dem färbenden Inhaltsstoffen Alkannan (C.I. 75520) und Alkannin (C.I. 75530) beziehungsweise Shikonin (C.I. 75535): 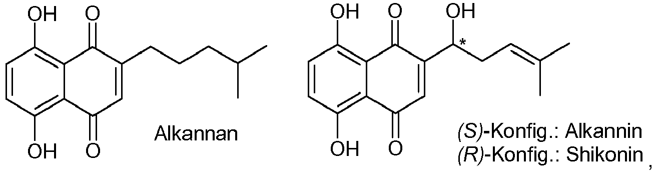 in Lapachoholz von *Tecoma ipé, Tecoma lapacho, Tecoma leucoxylon* oder *Tecoma achracea* (C.I. Natural Yellow 16) mit dem färbenden Inhaltsstoffen Lapachol (C.I. 75490), sowie untergeordnet Desoxylapachol, α-Lapachon, β-Lapachon, Melchinon I, Lapachononund Dehydro-α-lapachon: 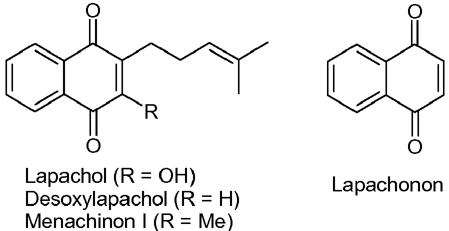 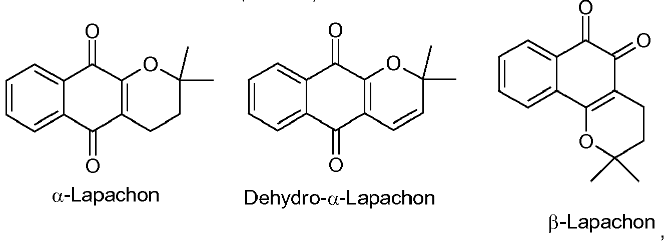
c) Anthrachinonfarbstoffe,
   wie beispielsweise in Krapp-Wurzeln (C.I. Natural Red 8) - *Rubia peregrina* und *Rubia tinctorum* mit den färbenden Inhaltsstoffen Purpurin (C.I. 75410), Pseudopurpurin (C.I. 75420; C.I. 58220-snythetisch), Alizarin (C.I. 75330), Rubiadin (C.I. 75350) und Munjistin (C.I. 75370): 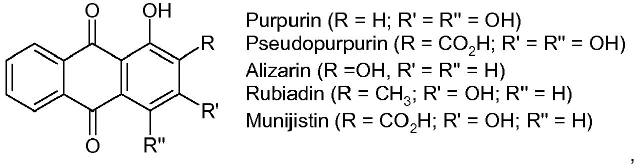 in den Wurzeln und Stengeln des Indischen Krapp (C.I. Natural Red 16) - *Rubia cordifolia* und *Rubia sikkimensis* mit den färbenden Inhaltsstoffen Purpurin (C.I. 75410), Pseudopurpurin (C.I. 75420; C.I. 58220-snythetisch), Alizarin (C.I. 75330), und Munjistin (C.I. 75370) sowie Nordamnacanthal, Physcion und Purpuroxanthin (C.I. 75340): 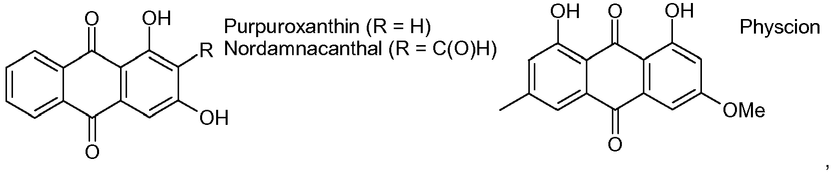 in Rharbarber-Wurzeln - *Rheum undulatum L., Rheum palmatum L.* und weitere Rheum-Spezies mit den färbenden Inhaltsstoffen Emodin, Chrystophanol (C.I. 75400; C.I. Natural Yellow 23), Aloeemodin, Physcion, Rhein, den Heterodianthronen Rhéidin A, B, C, Palmidin A, B, C, D und Sennidin C sowie davon abgeleitete Anthra- und Diglykoside: 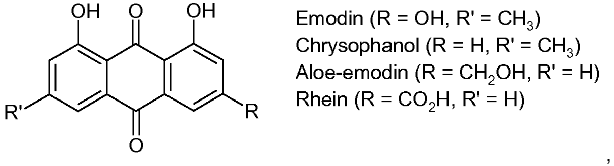 in den Wurzeln des Echten Labkraut - *Galium verum L.,* Gemeinen Labkraut - *Galium mollugo L.,* Klebkraut - *Galium aparine L.,* Waldmeister - *Galium odoratum* (C.I. Natural Red 14) mit den färbenden Inhaltsstoffen Purpurin (C.I. 75410), Pseudopurpurin (C.I. 75420; C.I. 58220-snythetisch), Alizarin (C.I. 75330), Rubiadin (C.I. 75350), Purpuroxanthin (C.I. 75340), Lucidin, Alizarin-1-methylether, Alizarin-2-methylether, 2-Hydroxyanthrachinon und 1-Hydroxy-2-methylanthrachinon: 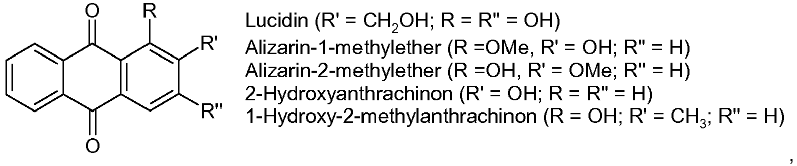 in Färbermeisterwurzel (C.I. Natural Red 13) - *Asperula tinctoria* mit den färbenden Inhaltsstoffen Purpurin (C.I. 75410), Pseudopurpurin (C.I. 75420; C.I. 58220-snythetisch) und Alizarin (C.I. 75330),
   in Rinde des Faulbaum - *Rhamnus fragula L. tinctoria* mit den färbenden Inhaltsstoffen Emodin, 4 Emodin-Glukoside: Glucofragulin A, Glucofrangulin B, Frangulin A, Frangulin B, Physcion und Chrysophanol (C.I. 75400): 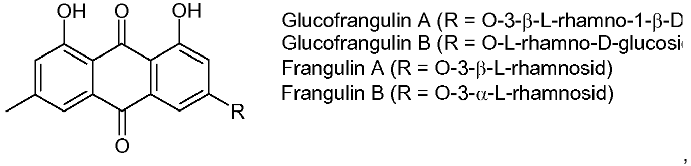
d) Indigoide Farbstoffe,
   wie beispielsweise in den Blättern und teilweise Stengeln des Indischem Indigo - *Indigofera tinctoria (Indigofera argentea),* Färberwaid - *Isatis tinctoria,* Färberoleander - *Wrightia tinctoria* und Wildem Indigo - *Baptisia tinctoria* (C.I. Natural Blue 1) mit den Farbstoffvorprodukten Isatan B und Indican, welche durch einen Gärungsprozess oxidativ in den eigentlichen Farbstoff Indigo (C.I. 75780; C.I. 73000) sowie untergeordnet Indirubin (C.I. 75790; C.I. 73200) und Isatin überführt werden: 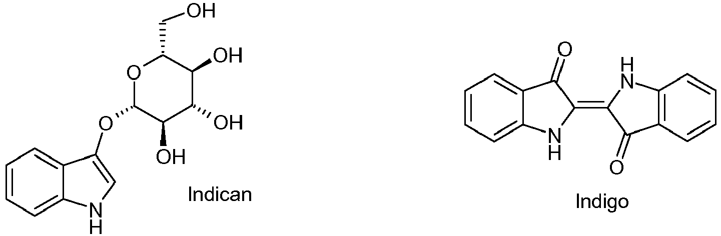 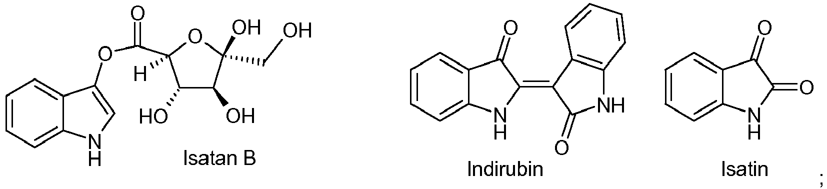 unter anderem unter den Namen Indigofera folium oder Henna, schwarz als Handelsprodukt, zumeist in Kombination mit Extrakten von *Lawsonia Inermis,* erhältlich,
e) Flavonoidfarbstoffe,
   wie beispielsweise in den Blütenköpfen von Römischer Kamille - *Chamaemelum nobile* (C.I. Natural Yellow 1) und Deutscher Kamille - *Chamomilla recutica* mit den färbenden Inhaltsstoffen Apigenin (C.I. 75580; C.I. Natural Yellow 1,2), Rutin (C.I. 75730; Natural Yellow 10), sowie untergeordnet Luteolin (C.I. 75590), Kämpferol (C.I. 75460, C.I. Natural Yellow 13, 10) und Quercetin (C.I. 75670; C.I. Natural Yellow 10,13; C.I. Natural Red 1) und davon abgeleitete Glukoside: 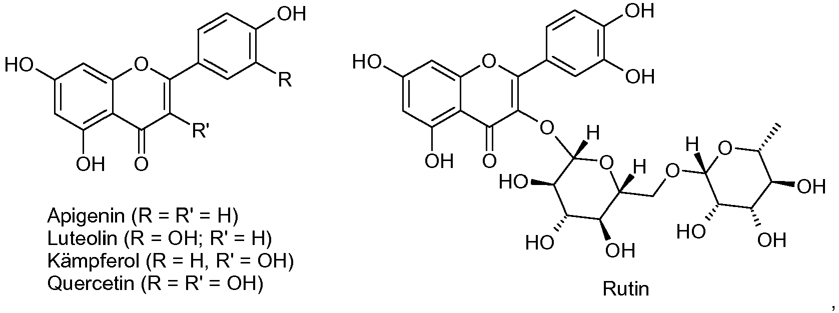 in den Blättern und Stengeln von Salbei - *Salvia tribola L.* mit den färbenden Inhaltsstoffen Luteolin (C.I. 75590) und Salvigenin: 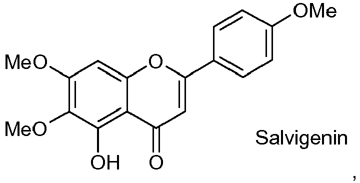 in den Blättern und Stengeln von Rosmarin - *Rosmarinus Officinalis* mit den färbenden Inhaltsstoffen Luteolin (C.I. 75590) und Apigenin (C.I. 75580; C.I. Natural Yellow 1,2) sowie weiteren Flavonoiden,
f) Neoflavanoidfarbstoffe,
   wie beispielsweise in Blauholz (C.I. Natural Black 1), Kernholz von *Haematoxylum campechianum L*. mit dem färbenden Flavinoid Quercetin und dem Farbstoffvorprodukt Hämatoxylin, welches oxidativ in den Farbstoff Hämatein (C.I. 75290, C.I. Natural Black 1 und 2) umgewandelt wird: 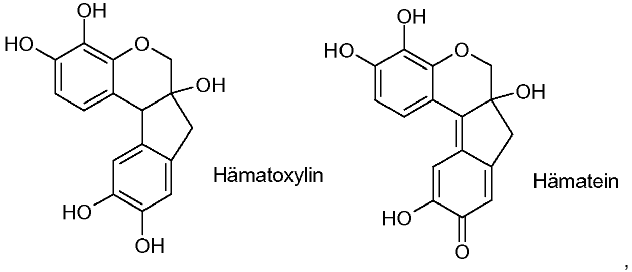 in Sappanholz - *Caesalpinia sappan L.,* Brazilienholz - *Caesalpinia brasiliensis L.,* Nicaraguaholz *- Caesalpinia echinata,* Pernambunkholz - *Caesalpinia crista L.,* Brasilietteholz - *Haematoxylum braziletto*, jeweils als Kernholz (C.I. Natural Red 24), mit dem Farbstoffvorprodukt Brasilin, welches oxidativ in den Farbstoff Brasilein (C.I. 75280, C.I. Natural Red 24) umgewandelt wird: 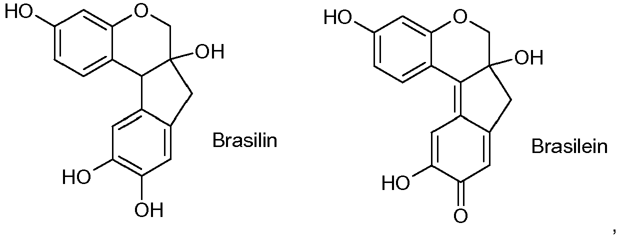
g) unlösliche Rotholz-Farbstoffe,
   wie beispielsweise in Rotem Sandelholz, Kernholz von *Pterocarpus santalinus L.,* Narraholz - *Pterocarpus indicus L.,* Barholz - *Pterocarpus soyauxii L.,* Camholz - *Bahia nitida* (C.I. Natural Red 22) mit den färbenden Inhaltsstoffen Santalin A, Santalin B, Tetra-O-methyl-santarubin, Hompterocarpin, Pterocarpin, Maackiain, den Isoflavioniden Formononetin und Santal, sowie dem Chalkon Isoliquiritigenin und dem Flavon Liquiritigenin: 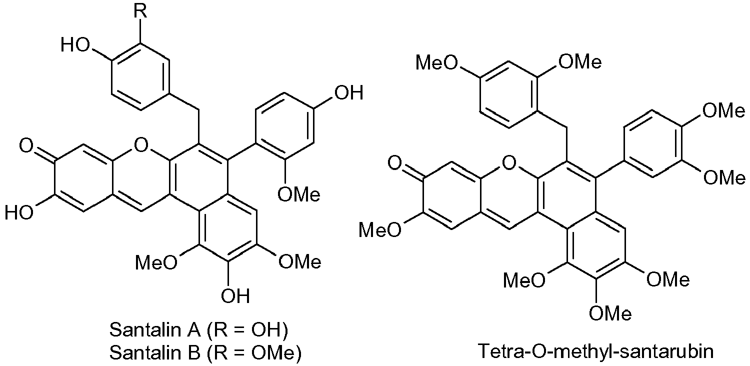 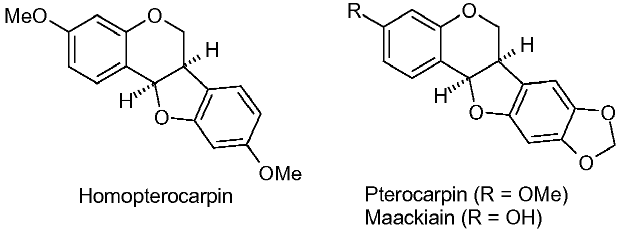 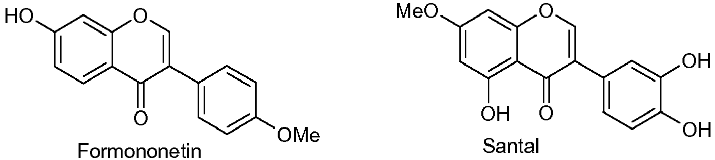 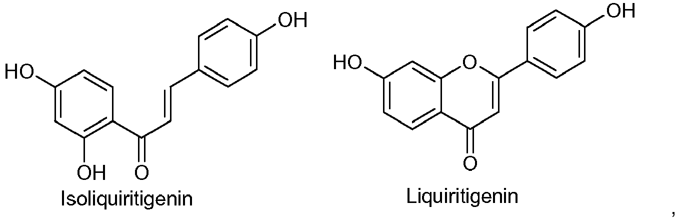
h) Betalainfarbstoffe
   wie beispielsweise in Rote Beete Wurzeln - *Beta vulgaris* mit den färbenden Hauptinhaltsstoffen Betanidin und Praebetanin sowie untergeordnet weiteren Betacyaninen, Betaxanthinen, Vulgaxanthinen, Anthocyanen und Eisen- und Kupfer-Komplexen: 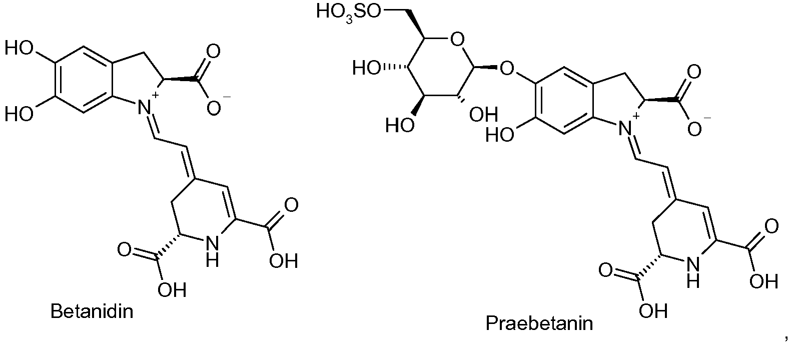
i) Gallotanninfarbstoffe
   wie beispielsweise in der Rinde von Stiel- oder Sommereiche - *Quercus robur L.* mit den färbenden Hauptinhaltsstoffen aus der Gruppe der Gallotannine und Ellagentannine (Glykoside der Gallussäure beziehungsweise der Ellagsäure; C.I. 75270): 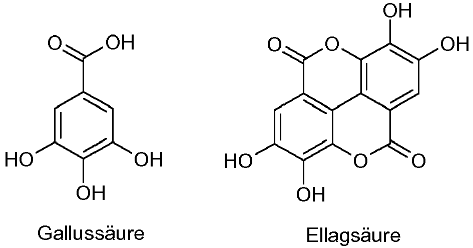
j) Farbstoffe aus kondensierten Gerbstoffen,
   wie beispielsweise in Catechu (C.I. Natural Brown 3), eine Mischung aus Extrakten von Färbepflanzen, vorzugsweise *Acacia catechu, Areca catechu* und *Uncaria gambier,* mit einem hohen Anteil von kondensierten Gerbstoffen (,Proanthocyanidine') vom Catechin-Typ: 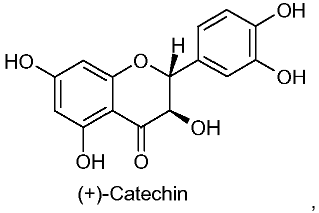 wie in den Blättern von grünem und schwarzem (= fermentiertem grünen) Tee - *Camellia sinensis,* mit den Inhaltsstoffen Gallussäure, *m*-Digallussäure, Catechin, Glykosiden von Flavonoiden wie, unter anderen, Luteolin, Apigenin, Kämpferol, Quercetin, Myriecetin, sowie Thearubingenine (,Proanthocyanidine'), Theaflavin und Isotheaflavin:

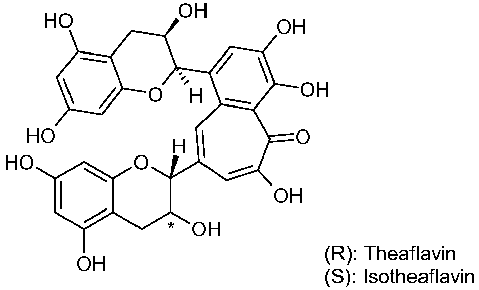

Insbesondere enthalten die erfindungsgemäßen kosmetischen Mittel mindestens einen Naturfarbstoff, der ausgewählt wird aus der Gruppe, die Diaryloylmethanfarbstoffe, Naphthochinonfarbstoffe, Flavonoidfarbstoffe, Anthrachinonfarbstoffe, Betalainfarbstoffe, Gallotanninfarbstoffe und indigoide Farbstoffe umfasst.
Erfindungsgemäß besonders bevorzugte Naturfarbstoffe sind solche, die aus einer Pflanze erhalten wurde, die aus der Gruppe ausgewählt wird, die gebildet wird aus Indigofera tinctoria, Curcuma longa, Lawsonia inermis, Chamomilla recutita, Quercus robur, Rosmarinus officinalis, Rheum undulatum und Beta vulgaris.

Ganz besonders bevorzugt ist dabei die Gruppe, die gebildet wird aus Diaryloylmethanfarbstoffen, Naphthochinonfarbstoffen und indigoiden Farbstoffen.
Ganz besonders bevorzugt sind in den erfindungsgemäßen kosmetischen Mitteln Naturfarbstoffe von Pflanzen enthalten, die ausgewählt sind aus der Gruppe, zusammengesetzt aus Curcuma longa (Kurkuma; C.I. Natural Yellow 3), Lawsonia inermis (Henna, rot; C.I. Natural Orange 6) und Indigofera tinctoria (Henna, schwarz; C.I. Natural Blue 1).

Die Naturfarbstoffe werden jeweils bevorzugt in einer Menge von 0,001 bis 20 Gew.-%, bezogen auf die gesamte Anwendungszubereitung, eingesetzt. Die Gesamtmenge an Naturfarbstoffen beträgt vorzugsweise höchstens 20 Gew.-%.

Die erfindungsgemäßen Färbemittel können weiterhin alle für solche Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel R¹O-(Z)ₓ. Diese Verbindungen sind durch die folgenden Parameter gekennzeichnet.

Der Alkylrest R¹ enthält 6 bis 22 Kohlenstoffatome und kann sowohl linear als auch verzweigt sein. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest R¹ enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Die Alkylglykoside können neben ihrer Tensidwirkung auch dazu dienen, die Fixierung von Duftkomponenten auf dem Haar zu verbessern. Der Fachmann wird also für den Fall, dass eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyldimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine - COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Erfindungsgemäß werden als kationische Tenside insbesondere solche vom Typ der quartären Ammoniumverbindungen, der Esterquats und der Amidoamine eingesetzt.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen.

Weitere erfindungsgemäß verwendbare kationische Tenside stellen die quaternisierten Proteinhydrolysate dar.

Erfindungsgemäß ebenfalls geeignet sind kationische Silikonöle.

Ein Beispiel für ein als kationisches Tensid einsetzbares quaternäres Zuckerderivat stellt das Handelsprodukt Glucquat^{®}100 dar, gemäß INCI-Nomenklatur ein "Lauryl Methyl Gluceth-10 Hydroxypropyl Dimonium Chloride".

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.
- Ferner können die erfindungsgemäßen Färbemittel weitere Wirk-, Hilfs- und Zusatzstoffe enthalten

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Die erfindungsgemäßen Mittel enthalten die Farbstoffvorprodukte sowie die erfindungsgemäße Wirkstoffkombination bevorzugt in einem geeigneten wässrigen, alkoholischen oder wässrig-alkoholischen Träger. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind.

Es ist aber auch denkbar, die Farbstoffvorprodukte in eine pulverförmige oder auch Tablettenförmige Formulierung zu integrieren.

Unter wässrig-alkoholischen Lösungen sind im Sinne der vorliegenden Erfindung wässrige Lösungen enthaltend 3 bis 70 Gew.-% eines C₁-C₄-Alkohols, insbesondere Ethanol beziehungsweise Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Die eigentliche oxidative Färbung der Fasern kann grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Als Oxidationsmittel kommen Persulfate, Chlorite und insbesondere Wasserstoffperoxid oder dessen Anlagerungsprodukte an Harnstoff, Melamin sowie Natriumborat in Frage. Erfindungsgemäß kann aber das Oxidationsfärbemittel auch zusammen mit einem Katalysator auf das Haar aufgebracht werden, der die Oxidation der Farbstoffvorprodukte, z.B. durch Luftsauerstoff, aktiviert. Solche Katalysatoren sind z.B. Metallionen, Iodide, Chinone oder bestimmte Enzyme.

Geeignete Metallionen sind beispielsweise Zn²⁺, Cu²⁺, Fe²⁺, Fe³⁺, Mn²⁺, Mn⁴⁺, Li⁺, Mg²⁺, Ca²⁺ und Al³⁺. Besonders geeignet sind dabei Zn²⁺, Cu²⁺ und Mn²⁺. Die Metallionen können prinzipiell in der Form eines beliebigen, physiologisch verträglichen Salzes oder in Form einer Komplexverbindung eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflusst werden.

Geeignete Enzyme sind z.B. Peroxidasen, die die Wirkung geringer Mengen an Wasserstoffperoxid deutlich verstärken können. Weiterhin sind solche Enzyme erfindungsgemäß geeignet, die mit Hilfe von Luftsauerstoff die Oxidationsfarbstoffvorprodukte direkt oxidieren, wie beispielsweise die Laccasen, oder *in situ* geringe Mengen Wasserstoffperoxid erzeugen und auf diese Weise die Oxidation der Farbstoffvorprodukte biokatalytisch aktivieren. Besonders geeignete Katalysatoren für die Oxidation der Farbstoffvorläufer sind die sogenannten 2-Elektronen-Oxidoreduktasen in Kombination mit den dafür spezifischen Substraten, z.B.
- Pyranose-Oxidase und z.B. D-Glucose oder Galactose,
- Glucose-Oxidase und D-Glucose,
- Glycerin-Oxidase und Glycerin,
- Pyruvat-Oxidase und Benztraubensäure oder deren Salze,
- Alkohol-Oxidase und Alkohol (MeOH, EtOH),
- Lactat-Oxidase und Milchsäure und deren Salze,
- Tyrosinase-Oxidase und Tyrosin,
- Uricase und Harnsäure oder deren Salze,
- Cholinoxidase und Cholin,
- Aminosäure-Oxidase und Aminosäuren.

Das eigentliche Haarfärbemittel wird zweckmäßigerweise unmittelbar vor der Anwendung durch Mischung der Zubereitung des Oxidationsmittels mit der Zubereitung, enthaltend die Farbstoffvorprodukte, hergestellt. Das dabei entstehende gebrauchsfertige Haarfärbepräparat sollte bevorzugt einen pH-Wert im Bereich von 6 bis 12 aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Insbesondere bei schwer färbbarem Haar kann die Zubereitung mit den Farbstoffvorprodukten aber auch ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert. Bei dieser Ausführungsform wird gemäß einer ersten Variante, bei der das vorherige Aufbringen der Farbstoffvorprodukte eine bessere Penetration in das Haar bewirken soll, das entsprechende Mittel auf einen pH-Wert von etwa 4 bis 7 eingestellt. Gemäß einer zweiten Variante wird zunächst eine Luftoxidation angestrebt, wobei das aufgebrachte Mittel bevorzugt einen pH-Wert von 7 bis 10 aufweist. Bei der anschließenden beschleunigten Nachoxidation kann die Verwendung von sauer eingestellten Peroxidisulfat-Lösungen als Oxidationsmittel bevorzugt sein.

Ein weiterer Gegenstand der vorliegenden Erfindung ist die Verwendung eines der erfindungsgemäßen Mittel zur Färbung keratinischer Fasern.

Ein dritter Gegenstand der vorliegenden Erfindung ist ein Verfahren zur Färbung keratinischer Fasern, insbesondere menschlicher Haare, bei dem ein Mittel A, enthaltend mindestens ein Farbstoffvorprodukt, mit einem Mittel B, enthaltend mindestens ein Oxidationsmittel, unmittelbar vor der Anwendung gründlich vermischt wird, die resultierende Anwendungszubereitung auf die Fasern aufgebracht wird und nach einer Einwirkzeit von 1 bis 60 Minuten wieder abgespült wird, wobei das Mittel A und/oder die resultierende Anwendungszubereitung die erfindungsgemäße Wirkstoffkombination enthält.

Im Rahmen dieses Verfahrens hat es sich als besonders vorteilhaft erwiesen, wenn die Fasern anschließend mit einer reinigenden und/oder pflegenden Zubereitung behandelt werden, enthaltend eine Wirkstoffkombination aus
(a) mindestens einer basischen Aminosäure,
(b) Pyrrolidoncarbonsäure oder einer ihrer physiologisch verträglichen Salze,
wobei auch diese Zubereitung im Wesentlichen frei von sauren Aminosäuren ist.

Ein vierter Gegenstand der vorliegenden Erfindung ist ein Kit zur Färbung keratinischer Fasern, enthaltend zumindest eine erste Verpackungseinheit, enthaltend ein Mittel A, enthaltend mindestens ein Farbstoffvorprodukt, und eine zweite Verpackungseinheit, enthaltend ein Mittel B, enthaltend mindestens ein Oxidationsmittel, wobei das Mittel A und/oder die durch Mischung der Mittel A und B erhältliche Anwendungszubereitung eine erfindungsgemäß Wirkstoffkombination enthält.

Im Rahmen dieser Ausführungsform hat es sich als besonders vorteilhaft erwiesen, wenn das Kit weiterhin eine dritte Verpackungseinheit aufweist, die eine reinigende und/oder pflegende Zubereitung enthält, die eine Wirkstoffkombination aus
(a) mindestens einer basischen Aminosäure,
(b) Pyrrolidoncarbonsäure oder einer ihrer physiologisch verträglichen Salze,
wobei auch diese Zubereitung im Wesentlichen frei von sauren Aminosäuren ist.

Bezüglich weiterer bevorzugter Ausführungsformen der Gegenstände zwei bis vier der vorliegenden Erfindung gilt mutatis mutandis das zu den erfindungsgemäßen Mitteln Gesagte. Dies gilt insbesondere auch für die bevorzugten Ausführungsformen des Nachbehandlungsmittels.

Die folgenden Beispiele sollen den Gegenstand der vorliegenden Erfindung weiter erläutern ohne diesen in irgendeiner Art zu beschränken.

### Ausführungsbeispiele

Die folgenden Mengenangaben verstehen sich - soweit nichts anderers vermerkt ist - jeweils in Gewichtsprozent.
1. Es wurden die folgenden Färbecremes hergestellt: 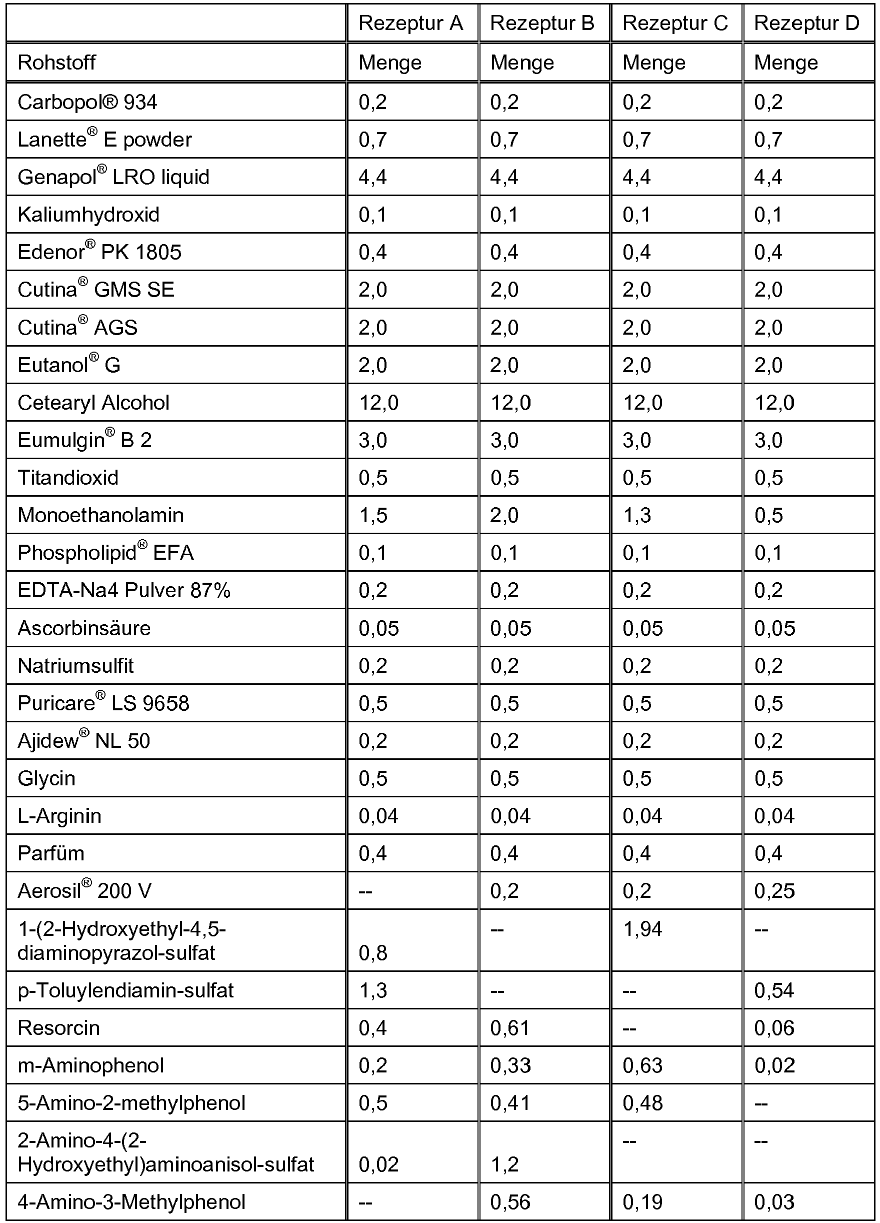 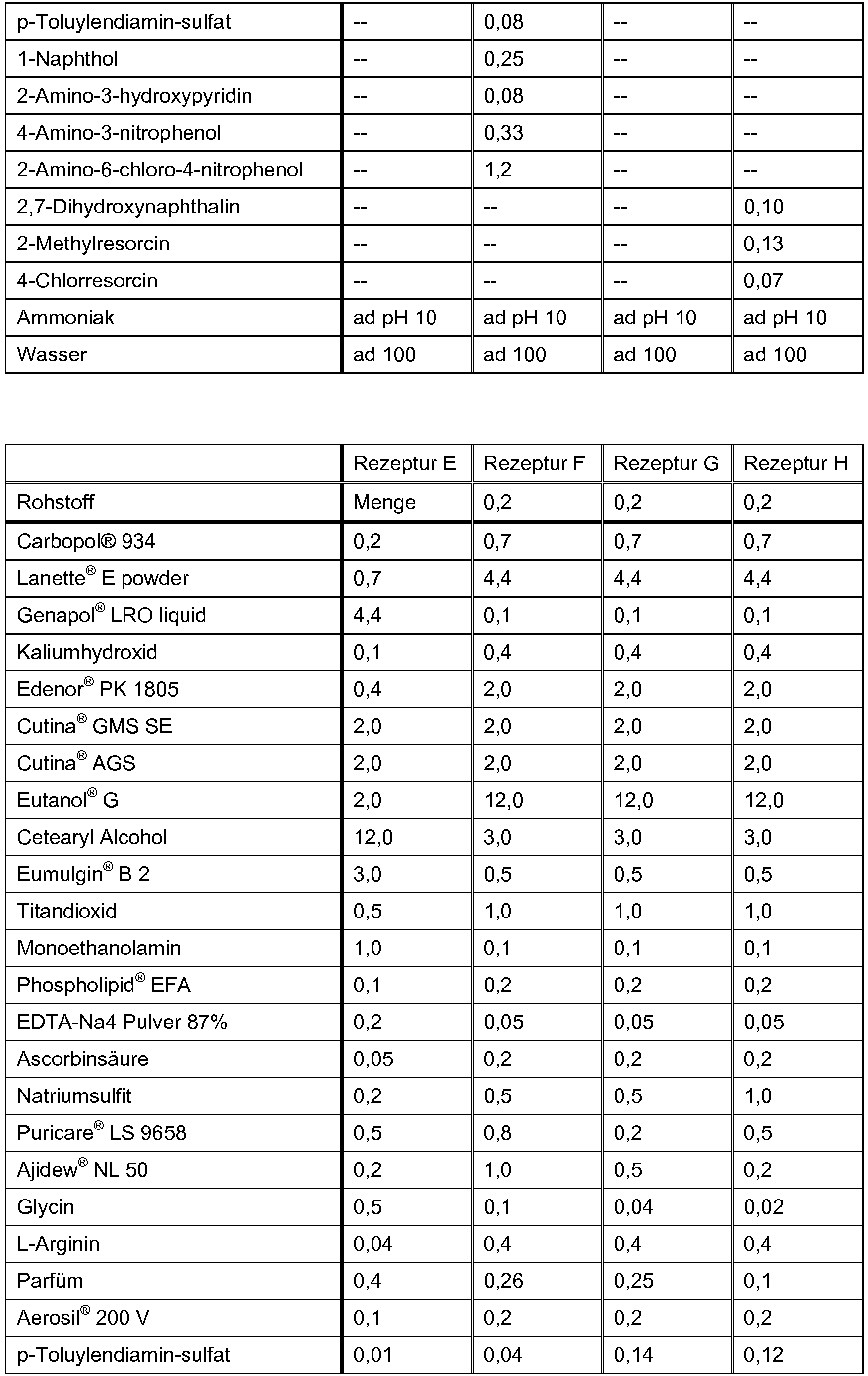 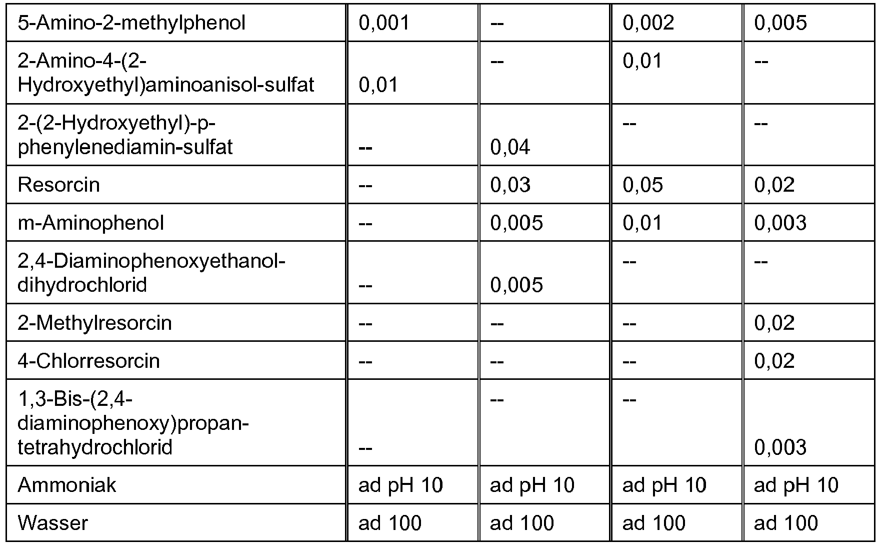
2. Es wurden die folgenden Oxidationsmittelzubereitungen hergestellt:

| | Rezeptur 1 | Rezeptur 2 | Rezeptur 3 |
|---|---|---|---|
| Rohstoff | Menge | Menge | Menge |
| Natriumbenzoat | 0,04 | 0,04 | 0,04 |
| Dipicolinsäure | 0,1 | 0,1 | 0,1 |
| Dinatriumpyrophosphat | 0,1 | 0,1 | 0,1 |
| Kaliumhydroxid 50% | 0,2 | 0,2 | 0,2 |
| 1,2-Propandiol | 0,5 | 0,5 | 0,5 |
| Turpinal^{®} SL | 0,3 | 0,3 | 0,3 |
| Paraffinum Liquidum | 0,3 | 0,3 | 0,3 |
| Genamin^{®} STAC | 0,2 | 0,2 | 0,2 |
| Cetearyl Alcohol | 3,4 | 3,4 | 3,4 |
| Ceteareth-20 | 1,0 | 1,0 | 1,0 |
| Wasserstoffperoxid 50 % | 12,2 | 6,2 | 18,2 |
| Parfüm | 0,05 | 0,05 | 0,05 |
| Wasser | ad 100 | ad 100 | ad 100 |

*Verzeichnis der eingesetzten Handelsprodukte*
- Aerosil^{®} 200 V: Kieselsäure (INCI-Bezeichnung: Silica) (Degussa)
- Ajidew^{®} NL50: Natriumsalz der Pyrrolidoncarbonsäure (ca. 48-52% Aktivsubstanzgehalt; INCI-Bezeichnung: Sodium PCA) (Ajinomoto)
- Carbopol^{®} 934: Polyarcylsäure (INCI-Bezeichnung: Carbomer) (Noveon)
- Cutina^{®} AGS: Ethylenglykoldistearat (INCI-Bezeichnung: Glycol Distearate) (Cognis)
- Cutina^{®} GMS-SE: INCI-Bezeichnung: Glyceryl Stearate SE (Cognis)
- Edenor^{®} PK 1805: Ölsäure (INCI-Bezeichnung: Oleic Acid) (Cognis)
- Eumulgin^{®} B2: Cetylstearylalkohol mit ca. 20 EO-Einheiten (INCI-Bezeichnung: Ceteareth-20) (Cognis)
- Genamin^{®} STAC: Trimethylstearylammoniumchlorid (ca. 80% Aktivsubstanz in Isopropanol; INCI-Bezeichnung: Steartrimonium Chloride) (Clariant)
- Genapol^{®} LRO liquid: Natriumlaurylethersulfat (ca. 27.5-28,5% Aktivsubstanzgehalt; INCI-Bezeichnung: Sodium lauryl ether sulfate) (Clariant)
- Lanette^{®} E: Fettalkoholsulfat-Natrium-Salz (ca. 90-96% Aktivsubstanzgehalt; INCI-Bezeichnung: Sodium Cetearyl Sulfate) (Cognis)
- Phopholipid^{®} EFA: (ca. 30% Festkörper in Wasser/Propylenglykol; INCI-Bezeichnung:Linoleamidopropyl PG-Dimonium Chloride Phosphate) (Uniqema)
- Puricare^{®} LS 9658: Moringa Pterygosperma Extrakt, in Wasser/Glycerin (0,5 %ig, INCI-Bezeichnung: Water, Glycerine, Moringa Pterygosperma Seed Extract) (Cognis)
- Turpinal^{®} SL: 1-Hydroxyethan-1,1-diphosphonsäure (ca. 58 - 61% Aktivsubstanzgehalt; INCI-Bezeichnung: Etidronic Acid, Aqua (Water)) (Solutia)

3. Anwendung
Die Färbecremes A, B, C und D wurden jeweils im Mengenverhältnis 1:1 mit der Oxidationsmittelzubereitung 1 angemischt und die resultierende Mischung auf trockenes Yakhaar (Büffelbauchhaar, Fa. Hohenschildt & Gottschalk, Berlin) aufgebracht. Nach 10 min Einwirkzeit wurde die Färbemischung abgepült und das Haar getrocknet. Es wurden die folgenden, intensiven Färbungen der Haare erzielt:
Färbecreme A: Bordeauxbraun,
Färbecreme B : Kupferbraun,
Färbecreme C : Intensivrot,
Färbecreme D : Hellblond.

Durch Zugabe von 0,1 % Asparaginsäure verloren insbesondere die Modetöne A, B und C an Leuchtkraft und Intensität.
Außerdem wurde die Grauabdeckung der unterschiedlichen Färbemittel mit Hilfe von farbmetrischen Messungen im CIE-L,a,b-System bestimmt. Neben den oben beschriebenen Rezepturen wurden dabei Rezepturen verwendet, die die Rohstoffe Argining und Ajidew (Pyrrolidoncarbonsäure) nicht enthielten. Für diese nicht erfindungsgemäßen Rezepturen wurde darüber hinaus die Grauabdeckung nach 30 Minuten Einwirkzeit des Färbemittels ermittelt.

| Rezeptur | Graudeckung nach 10min Einwirkzeit | Graudeckung nach 30min Einwirkzeit |
|---|---|---|
| Färbecreme A | 95 % | n.b. |
| Färbecreme A ohne Arginin/Ajidew | 89 % | 93 % |
| Färbecreme B | 96 % | n.b. |
| Färbecreme B ohne Arginin/Ajidew | 91 % | 92 % |
| Färbecreme C | 83 % | n.b. |
| Färbecreme C ohne Arginin/Ajidew | 80 % | 80 % |

Den Daten kann entnommen werden, dass das Weglassen von Ajidew und/oder Arginin insbesondere bei den extremen Modetönen zu sichtbar geringeren Farbintensitäten und Graudeckungsleistungen der Färbemischungen führt.
Dieser Effekt konnte nicht einmal durch verlängerte Einwirkzeiten auf 30min kompensiert werden:
Die Färbecremes E, F, G und H wurden jeweils im Mengenverhältnis 1:1 mit der Oxidationsmittelzubereitung 3 angemischt, und die resultierende Mischung auf trockenes dunkelbondes Naturhaar (Fa. Kerling, Naturhaar 6-0) aufgebracht. Nach 10min Einwirkzeit wurden die Haare ausgespült und anschließend getrocknet. Es wurden Aufhellungen auf die Grundfarbtiefe Helllbond in den folgenden Farbrichtungen erzielt:
   Färbecreme E : neutral,
   Färbecreme F : cendre,
   Färbecreme G : matt,
   Färbecreme H : asch.

Das Weglassen von Arginin und Ajidew aus den Färbecremes führte zu einer deutlich geringeren Aufhellleistung. Ein Zusatz von 0,1 % Asparaginsäure führte zu unerwünschten Verschiebungen der Farbrichtungen in die gelbliche Richtung.

## Patentansprüche

1. Mittel zur oxidativen Färbung keratinischer Fasern, insbesondere menschlicher Haare, enthaltend in einem kosmetisch akzeptablen Träger mindestens ein Oxidationsfarbstoffvorprodukt sowie eine Wirkstoffkombination aus
(a) mindestens einer basischen Aminosäure,
(b) Pyrrolidoncarbonsäure oder einer ihrer physiologisch verträglichen Salze,
**dadurch gekennzeichnet, dass** das Mittel im Wesentlichen frei von sauren Aminosäuren ist.

2. Mittel nach Anspruch 1, **dadurch gekennzeichnet, dass** es weiterhin mindestens eine neutrale Aminosäure enthält.

3. Mittel nach Anspruch 2, **dadurch gekennzeichnet, dass** mindestens eine aliphatische neutrale Aminosäure enthält.

4. Mittel nach einem der Ansprüche 2 oder 3, **dadurch gekennzeichnet, dass** das Massenverhältnis von neutraler Aminosäure (nA) zu basischer Aminosäure (bA) nA : bA zwischen 20 und 1 liegt.

5. Mittel nach einem der Ansprüche 1 bis 4, **dadurch gekennzeichnet, dass** der Gehalt an sauren Aminosäuren geringer als 0,05Gew.-%, vorzugsweise geringer als 0,01Gew.-% liegt.

6. Mittel nach einem der Ansprüche 1 bis 5, **dadurch gekennzeichnet, dass** die basische Aminosäure in einer Menge von 0,001 bis 0,2 Gew.-%, vorzugsweise von 0,01 bis 0,1 Gew.-%, bezogen auf die anwendungsbereite Mischung enthalten ist.

7. Mittel nach einem der Ansprüche 1 bis 6, **dadurch gekennzeichnet, dass** es weiterhin mindestens einen Pflanzenextrakt enthält.

8. Mittel nach Anspruch 7, **dadurch gekennzeichnet, dass** der Pflanzenextrakt aus einer Pflanze gewonnen wird, die in tropischen und subtropischen Gebieten der Erde kultiviert wird.

9. Mittel nach Anspruch 8, **dadurch gekennzeichnet, dass** der Pflanzenextrakt aus Bestandteilen von Pflanzen der Gattung Moringa, vorzugsweise der Gattung Moringa Oleifera gewonnen wird.

10. Mittel nach einem der Ansprüche 1 bis 9, **dadurch gekennzeichnet, dass** als Alkalisierungsmittel ein organisches Amin, ausgewählt aus Monoethanolamin, Monoisopropanolamin, 2-Amino-2-methyl-propanol, 2-Amino-2-methyl-1,3-propandiol, 2-Amino-2-ethyl-1,3-propandiol, 2-Amino-2-methylbutanol und Triethanolamin, enthalten ist.

11. Verwendung eines Mittels nach einem der Ansprüche 1 bis 10 zur Färbung keratinischer Fasern.

12. Verfahren zur Färbung keratinischer Fasern, insbesondere menschlicher Haare, bei dem ein Mittel A, enthaltend mindestens ein Farbstoffvorprodukt, mit einem Mittel B, enthaltend mindestens ein Oxidationsmittel, unmittelbar vor der Anwendung gründlich vermischt wird, die resultierende Anwendungszubereitung auf die Fasern aufgebracht wird und nach einer Einwirkzeit von 1 bis 60 Minuten wieder abgespült wird, **dadurch gekennzeichnet, dass** das Mittel A und/oder die resultierende Anwendungszubereitung die Wirkstoffkombination gemäß einem der Ansprüche 1 bis 10 enthält.

13. Verfahren nach Anspruch 12, bei dem die Fasern anschließend mit einer reinigenden und/oder pflegenden Zubereitung behandelt werden, enthaltend eine Wirkstoffkombination aus
(a) mindestens einer basischen Aminosäure,
(b) Pyrrolidoncarbonsäure oder einer ihrer physiologisch verträglichen Salze,
**dadurch gekennzeichnet, dass** die Zubereitung im Wesentlichen frei von sauren Aminosäuren ist.

14. Kit zur Färbung keratinischer Fasern, enthaltend zumindest eine erste Verpackungseinheit, enthaltend ein Mittel A, enthaltend mindestens ein Farbstoffvorprodukt, und eine zweite Verpackungseinheit, enthaltend ein Mittel B, enthaltend mindestens ein Oxidationsmittel, **dadurch gekennzeichnet, dass** das Mittel A und/oder die durch Mischung der Mittel A und B erhältliche Anwendungszubereitung eine Wirkstoffkombination gemäß einem der Ansprüche 1 bis 10 enthält.

15. Kit nach Anspruch 13, **dadurch gekennzeichnet, dass** es weiterhin eine dritte Verpackungseinheit aufweist, die eine reinigende und/oder pflegende Zubereitung enthält, die eine Wirkstoffkombination aus
(a) mindestens einer basischen Aminosäure,
(b) Pyrrolidoncarbonsäure oder einer ihrer physiologisch verträglichen Salze,
enthält, **dadurch gekennzeichnet, dass** die Zubereitung im Wesentlichen frei von sauren Aminosäuren ist.

## Claims

1. An agent for oxidatively dyeing keratin fibers, in particular human hair, containing, in a cosmetically acceptable carrier, at least one oxidation dye precursor and an active ingredient combination of
(a) at least one basic amino acid,
(b) pyrrolidone carboxylic acid or one of the physiologically acceptable salt thereof, **characterized in that** the agent is substantially free of acidic amino acids.

2. The agent according to claim 1, **characterized in that** it also contains at least one neutral amino acid.

3. The agent according to claim 2, **characterized in that** it contains at least one aliphatic neutral amino acid.

4. The agent according to one of claims 2 or 3, **characterized in that** the mass ratio of the neutral amino acid (nA) to the basic amino acid (bA) (nA : bA) is between 20 and 1.

5. The agent according to one of claims 1 to 4, **characterized in that** the content of acidic amino acids is less than 0.05 wt.%, preferably less than 0.01 wt.%.

6. The agent according to one of claims 1 to 5, **characterized in that** it contains the basic amino acid in an amount of from 0.001 to 0.2 wt.%, preferably from 0.01 to 0.1 wt.%, based on the ready-to-use mixture.

7. The agent according to one of claims 1 to 6, **characterized in that** it additionally contains at least one plant extract.

8. The agent according to claim 7, **characterized in that** the plant extract is obtained from a plant that is cultivated in tropical and subtropical regions of the Earth.

9. The agent according to claim 8, **characterized in that** the plant extract is obtained from components of plants of the genus *Moringa*, preferably from the genus *Moringa oleifera.*

10. The agent according to one of claims 1 to 9, **characterized in that** it contains an organic amine selected from monoethanolamine, monoisopropanolamine, 2-amino-2-methyl-propanol, 2-amino-2-methyl-1,3-propanediol, 2-amino-2-ethyl-1,3-propanediol, 2-amino-2-methylbutanol und triethanolamine as the alkalizing agent.

11. The use of an agent according to one of claims 1 to 10 for dyeing keratin fibers.

12. A method for dyeing keratin fibers, in particular human hair, in which, directly before use, an agent A containing at least one dye precursor is thoroughly mixed with an agent B containing at least one oxidizing agent, the resulting preparation for use is applied to the fibers and is rinsed off again after a reaction time of from 1 to 60 minutes, **characterized in that** the agent A and/or the resulting preparation for use contains the active ingredient combination according to one of claims 1 to 10.

13. The method according to claim 12, in which the fibers are then treated with a cleansing and/or caring preparation containing an active ingredient combination of
(a) at least one basic amino acid,
(b) pyrrolidone carboxylic acid or one of the physiologically acceptable salt thereof,
**characterized in that** the preparation is substantially free of acidic amino acids.

14. A kit for dyeing keratin fibers, containing at least one first packaging unit containing an agent A that contains at least one dye precursor, and one second packaging unit containing an agent B that contains at least one oxidizing agent, **characterized in that** the agent A and/or the preparation for use obtainable by mixing agents A and B contains an active ingredient combination according to one of claims 1 to 10.

15. The kit according to claim 13, **characterized in that** it also contains a third packaging unit that contains a cleansing and/or caring preparation which contains an active ingredient combination of
(a) at least one basic amino acid,
(b) pyrrolidone carboxylic acid or one of the physiologically acceptable salt thereof,
**characterized in that** the preparation is substantially free of acidic amino acids.

## Revendications

1. Produit de coloration oxydante de fibres de kératine, en particulier de cheveux humains, contenant, dans un vecteur cosmétiquement acceptable, au moins un précurseur de colorant d'oxydation ainsi qu'une combinaison de substances actives parmi :
a) au moins un acide aminé basique
b) de l'acide pyrrolidone carboxylique ou un de ses sels physiologiquement acceptables,
**caractérisé en ce que** le produit est essentiellement exempt d'acides aminés acides.

2. Produit selon la revendication 1, **caractérisé en ce qu'**il contient en outre au moins un acide aminé neutre.

3. Produit selon la revendication 2, **caractérisé en ce qu'**il contient au moins un acide aminé neutre aliphatique.

4. Produit selon la revendication 2 ou 3, **caractérisé en ce que** le rapport pondéral de l'acide aminé neutre (nA) à l'acide aminé basique (bA) nA:bA est entre 20 et 1.

5. Produit selon une des revendications 1 à 4, **caractérisé en ce que** la teneur en acides aminés acides est inférieure à 0,05 % en poids, de préférence inférieure à 0,01 % en poids.

6. Produit selon une des revendications 1 à 5, **caractérisé en ce que** l'acide aminé basique est contenu à hauteur de 0,001 à 0,2% en poids, de préférence de 0,01 à 0,1 % en poids, rapporté au mélange prêt à l'emploi.

7. Produit selon une des revendications 1 à 6, **caractérisé en ce qu'**il contient en outre au moins un extrait végétal.

8. Produit selon la revendication 7, **caractérisé en ce que** l'extrait végétal s'obtient à partir d'une plante cultivée dans les régions tropicales et subtropicales de la Terre.

9. Produit selon la revendication 8, **caractérisé en ce que** l'extrait végétal s'obtient à partir de composants de plantes de la famille *Moringa*, de préférence de l'espèce *Moringa oleifera.*

10. Produit selon une des revendications 1 à 9, **caractérisé en ce qu'**il contient comme produit d'alcalinisation une amine organique choisie parmi la monoéthanolamine, la monoisopropanolamine, le 2-amino-2-méthylpropanol, le 2-amino-2-méthyl-1,3-propandiol, le 2-amino-2-éthyl-1,3-propandiol, le 2-amino-2-méthylbutanol et la triéthanolamine.

11. Utilisation d'un produit selon une des revendications 1 à 10 pour colorer des fibres de kératine.

12. Procédé de coloration de fibres de kératine, en particulier de cheveux humains, au cours duquel on mélange intimement un produit A contenant au moins un précurseur de colorant avec un produit B contenant au moins un oxydant, immédiatement avant utilisation, on applique la préparation prête à l'emploi obtenue sur les fibres, et après une durée d'action de 1 à 60 minutes, on la rince, **caractérisé en ce que** le produit A et/ou la préparation prête à l'emploi obtenue contient la combinaison de substances active selon une des revendications 1 à 10.

13. Procédé selon la revendication 12, dans lequel les fibres sont ensuite traitées avec une préparation nettoyante et/ou de soin, contenant une combinaison de substances actives choisies parmi:
a) au moins un acide aminé basique
b) de l'acide pyrrolidone carboxylique ou un de ses sels physiologiquement acceptables,
**caractérisé en ce que** la préparation est essentiellement exempte d'acides aminés acides.

14. Kit de coloration de fibres de kératine, contenant au moins une première unité de conditionnement contenant un produit A contenant au moins un précurseur de colorant et une deuxième unité de conditionnement contenant un produit B contenant au moins un oxydant, **caractérisé en ce que** le produit A et/ou la préparation prête à l'emploi qu'on obtient par mélange des produits A et B contient une combinaison de substances actives selon une des revendications 1 à 10.

15. Kit selon la revendication 13, **caractérisé en ce qu'**il présente en outre une troisième unité de conditionnement contenant une préparation nettoyante et/ou de soin contenant une combinaison de substances actives parmi :
a) au moins un acide aminé basique
b) de l'acide pyrrolidone carboxylique ou un de ses sels physiologiquement acceptables,
**caractérisé en ce que** la préparation est essentiellement exempte d'acides aminés acides.
